# EUROPEAN PATENT APPLICATION

(11) **EP 4 516 799 A1**
(43) Date of publication of application: **05.03.2025**
(21) Application number: 23796432.5
(22) Date of filing: 26.04.2023
(51) Int. Cl.: C07K 1/06, C07K 1/02, C07K 1/18

(54) **METHOD FOR PRODUCING POLYPEPTIDE COMPOUND**

(30) Priority: 27.04.2022 JP 2022073475
(71) Applicant: Chubu University Educational Foundation, Kasugai-shi Aichi 487-8501 (JP)
(72) Inventor: YAMAMOTO, Hisashi, Kasugai-shi, Aichi 487-8501 (JP); HATTORI, Tomohiro, Kasugai-shi, Aichi 487-8501 (JP)
(74) Representative: Hasegawa, Kan
(86) International application number: PCT/JP2023/016457
(87) International publication number: WO 2023/210692

(57) **Abstract**

This method comprises: (i) a step for forming an amide bond between a T^{a}-substituted carboxyl group located on the right side in formula (R1) in an N-terminal-protected amino acid or peptide ester represented by formula (R1) and an amino group amino group located on the left side in formula (R2) in an amino acid or peptide represented by formula (R2) or an ester compound thereof to produce an N-terminal-protected peptide represented by formula (S1); (ii) a step for deprotecting the N-terminal of the compound represented by formula (S1) produced in step (i) to produce a peptide compound represented by formula (P1); and (iii) a step for repeatedly performing the steps (i) and (ii) using the compound represented by formula (P1) produced in step (ii) as the compound represented by formula (R2) in step (i) to extend a peptide chain by amidation. In formulae (R1), (R2), (S1) and (P1), each symbol is as defined in the claims.

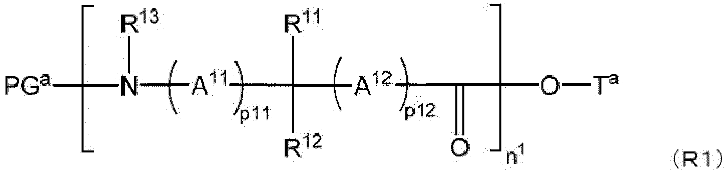

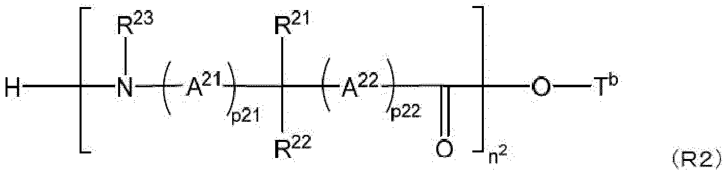

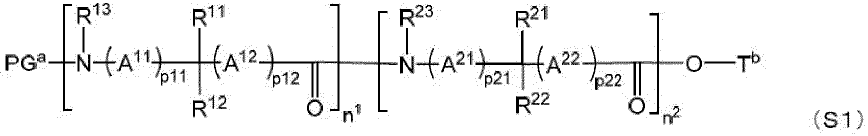

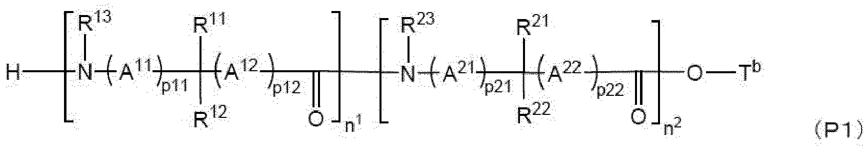

The symbols in formulae (R1), (R2), (S1), and (P1) each have the meanings defined in the claims.

## Description

### FIELD

The present invention relates to a novel method for producing polypeptide compounds.

### BACKGROUND

Conventionally, amide compounds represented by peptides have been used in a wide variety of fields, including pharmaceuticals, cosmetics, and functional foods. Development of synthetic methods thereof has been diligently pursued as an important research goal in synthetic chemistry (Non-Patent Literature 1 to 3). However, there are few truly effective catalysts or reaction agents other than carboxylic acid activators for the amidation reaction, which is the most important reaction in peptide synthesis. Therefore, it is unavoidable to use a reaction mode that forms by-products, and thus, peptide synthesis, which involves repeating multi-stage reactions, is extremely inefficient from the viewpoint of atom economy (atomic yield). There arises a large amount of by-product, and there are few effective purification means. As a result, the cost of disposal of by-products and purification constitutes most of the necessary costs for peptide synthesis and is the largest obstacle to development in this field.

In peptide synthesis, which uses amino acids or derivatives thereof as starting materials, it is desirable for the amidation reaction to proceed with high stereoselectivity. Enzyme reactions in the body are examples of highly stereoselective amidation reactions. For example, in the body, peptides are synthesized with extremely high stereoselectivity through sophisticated use of enzymes and hydrogen bonds. However, enzyme reactions are not suitable for mass production, requiring enormous financial and time costs when applied to synthetic chemistry.

In synthetic chemistry, amidation reactions using catalysts have been examined, but in conventional means, the amide bond is formed primarily through the method of activating carboxylic acid, such that racemization occurs quickly, whereby synthesizing a peptide with high stereoselectivity and efficiency is difficult.

According to conventional methods, it is very difficult to link an additional amino acid or derivative to a peptide comprising a plurality of amino acids or derivatives thereof (chemical ligation) or link two or more peptides via amide bonds. As an amidation method for ligation to such peptides, there are known methods for ligation by using an amino acid having a sulfur atom to utilize the high reactivity of the sulfur atom (Non-Patent Literature 4) and a method for ligation by synthesizing an amino acid hydroxyamine to utilize the high reactivity of the hydroxyamine (Non-Patent Literature 5). However, in the former method, it is difficult to synthesize amino acids having a sulfur atom, and in the latter method, hydroxyamine synthesis involving several steps is necessary. Both methods are time-consuming and costly and have a disadvantage in efficiency.

The present inventors have developed, as techniques for synthesizing an amide compound in a highly chemoselective manner: a method of amidating a carboxylic acid/ester compound having a hydroxy group at the β-position in the presence of a specific metal catalyst (Patent Literature 1); a method of using a hydroxyamino/imino compound as an amino acid precursor and amidating it in the presence of a specific metal catalyst, and then reducing them in the presence of a specific metal catalyst (Patent Literature 2); and a method of amidating a carboxylic acid/ester compound in the presence of specific metal catalyst (Patent Literature 3). The present inventors have also developed a technique for highly efficient and selective synthesis of peptides consisting of various amino acid residues by amide reaction of the carboxyl group of an N-terminal protected amino acid/peptide and the amino group of a C-terminal protected amino acid/peptide in the presence of a specific silylating agent and an optionally used Lewis acid catalyst, followed by deprotection (Patent Literature 4). The present inventors have further developed a method of synthesizing a peptide composed of various amino acid residues with a high efficiency and in a highly selective manner, by causing an amide reaction a carboxyl group of an amino acid or peptide whose N-terminal is either protected or unprotected and an amino group of an amino acid or peptide whose C-terminal is either protected or unprotected in the presence of a specific silylating agent, followed by deprotection (Patent Literatures 5 and 6), and a method for causing an amidation reaction using a Bronsted acid as a catalyst (Patent Literature 7), a novel silane-containing condensed ring dipeptide compound and a novel synthesis method for peptides using this compound (Patent Literature 8), as well as a novel synthesis method of peptides via regioselective C-N bond cleavage of lactam (Non-Patent Literature 6).

There is a growing need to establish a method for synthesizing peptides efficiently and quickly with a low cost. In particular, if such peptide synthesis could be carried out continuously in a flow reaction, it would be possible to omit the solvent treatment and compound purification stages of the reaction process, and it would also be possible to monitor the reaction intermediates as necessary, and even to carry out all operations automatically.

However, since the reactivity of peptide bond formation reactions is lower than that of normal amidation reactions, it is difficult to complete the reaction in the flow path. In addition, the use of a condensing agent, which is essential for conventional peptide synthesis reactions, can cause clogging in the flow path. Furthermore, there are cases where unwanted side reactions occur, such as when unreacted amino acids that have been mixed in at an earlier stage react unintentionally with the amino acids that are the target of the amidation reaction at a later stage. For this reason, it has been extremely difficult to carry out peptide synthesis reactions continuously using flow reactions.

### CITATION LIST

### Patent Literature

[Patent Literature 1] WO2017/204144 A
[Patent Literature 2] WO2018/199146 A
[Patent Literature 3] WO2018/199147 A
[Patent Literature 4] WO2019/208731 A
[Patent Literature 5] WO2021/085635 A
[Patent Literature 6] WO2021/085636 A
[Patent Literature 7] WO2021/149814 A
[Patent Literature 8] WO2022/190486 A

### Non-Patent Literature

[Non-Patent Literature 1] Chem. Rev., 2011, Vol.111, p.6557-6602
[Non-Patent Literature 2] Org. Process Res. Dev., 2016, Vol.20, No.2, p.140-177
[Non-Patent Literature 3] Chem. Rev., 2016, Vol.116, p.12029-12122
[Non-Patent Literature 4] Science, 1992, Vol.256, p.221-225
[Non-Patent Literature 5] Angew. Chem. Int. Ed., 2006, Vol.45, p.1248-1252
[Non-Patent Literature 6] Chem. Sci., (2022), Vol.13, pp.6309-6315
[Non-Patent Literature 7] Chem. Eur. J., 2019, Vol.25, p.15759-15764
[Non-Patent Literature 8] J. Med. Chem., 2001, Vol.44, p.3896-3903
[Non-Patent Literature 9] Org. Biomol. Chem., 2003, Vol.1, p.965-972
[Non-Patent Literature 10] J. Org. Chem., 1995, 60, 6, 1733-1740

### SUMMARY

### Problem to be Solved by the Invention

Against this background, there was a need for a method that could efficiently synthesize the desired peptide chain by continuously elongating the peptide chain through peptide bond reactions. The present invention has been made in view of this problem.

### Means for Solving the Problem

As a result of diligent study, the present inventors have found that an N-terminal protected amino acid or peptide ester (R1) with a C-terminal esterified with a monovalent aromatic hydrocarbon group or heterocyclic group T^{a} with an electron-withdrawing substituent is used as an electrophilic compound and mixed with a nucleophilic compound such as an amino acid or peptide or its ester (R2) to cause a peptide bond reaction, after which the N-terminal of the N-terminal protected amino acid or peptide (S1) obtained by the reaction is deprotected, and the deprotected amino acid or peptide (P1) is then subjected to a peptide bond reaction with another electrophilic compound (R1), whereby the peptide chain can be elongated by the peptide bond reaction continuously, making it possible to efficiently synthesize the desired peptide chain. Based on this finding, the present inventors have arrived at the present invention.

Thus, the present invention provides the following aspects.

### [Aspect 1]

A method for producing a polypeptide compound, comprising:
(i) forming an amide bond between the substituted carboxyl group on the right side of an N-terminal-protected amino acid ester or peptide ester compound represented by formula (R1) and the amino group on the left side of an amino acid or peptide or amino acid ester or peptide ester compound represented by formula (R2) to thereby produce an N-terminal-protected peptide compound represented by formula (S1);
(ii) deprotecting the N-terminal of the compound of formula (S1) from step (i) to thereby produce a peptide compound represented by formula (P1); and
(iii) repeating steps (i) and (ii) with using the compound of formula (P1) from step (ii) as a compound of formula (R2) of step (i) to thereby extend the peptide chain via amidation. 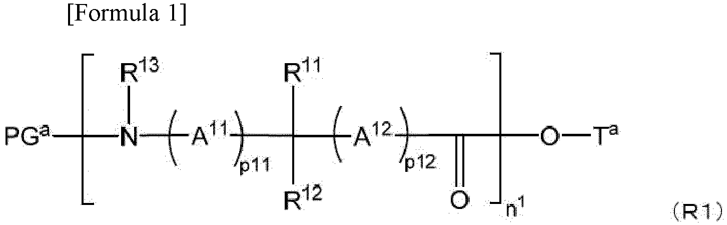 In formula (R1),
   T^{a} represents a monovalent aromatic hydrocarbon group or heterocyclic group having one or more electron-withdrawing substituents,
   PG^{a} represents a monovalent protecting group,
   R¹¹and R¹² each represent, independently of each other, a hydrogen atom, halogen atom, hydroxyl group, carboxyl group, nitro group, cyano group, or thiol group, or thiol group, or an amino group, monovalent aliphatic hydrocarbon group, monovalent aromatic hydrocarbon group, or monovalent heterocyclic group that may have one or more substituents,
   R¹³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
   R¹¹ and R¹³ may be bound to each other to form, together with the carbon atom to which R¹¹ binds and the nitrogen atom to which R¹³ binds, a heterocyclic group that may have one or more substituents,
   A¹¹ and A¹² each represent, independently of each other, a divalent aliphatic hydrocarbon group that may have one or more substituents having 1 to 3 carbon atoms,
   p11 and p12 each represent, independently of each other, 0 or 1, and
   n¹ represents an integer of equal to or greater than 1 corresponding to the number of the structure units parenthesized with [ ], provided that when n¹ is equal to or greater than 2, then the two or more structure units in [ ] may be either identical to each other or different from each other. 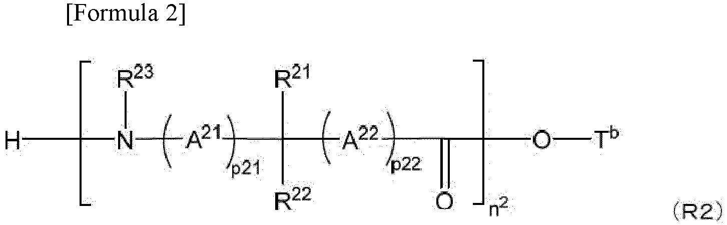
   In formula (R2),
      T^{b} represents a hydrogen atom or monovalent substituent,
      R²¹and R²² each represent, independently of each other, a hydrogen atom, halogen atom, hydroxyl group, carboxyl group, nitro group, cyano group, or thiol group, or thiol group, or an amino group, monovalent aliphatic hydrocarbon group, monovalent aromatic hydrocarbon group, or monovalent heterocyclic group that may have one or more substituents,
      R²³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
      R²¹ and R²³ may be bound to each other to form, together with the carbon atom to which R²¹ binds and the nitrogen atom to which R²³ binds, a heterocyclic group that may have one or more substituents,
      A²¹ and A²² each represent, independently of each other, a divalent aliphatic hydrocarbon group that may have one or more substituents having 1 to 3 carbon atoms,
      p21 and p22 each represent, independently of each other, 0 or 1, and
      n² represents an integer of equal to or greater than 1 corresponding to the number of the structure units parenthesized with [ ], provided that when n² is equal to or greater than 2, then the two or more structure units in [ ] may be either identical to each other or different from each other. 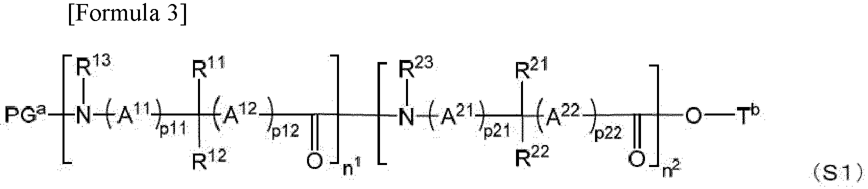
      In formula (S1),
         PG^{a}, R¹¹, R¹², R¹³, A¹¹, A¹², p11, p12, and n¹ each represent the same definitions as those of the same symbols in general formula (R1), and
         R²¹, R²², R²³, A²¹, A²², p21, p22, n², and T^{b} each represent the same definitions as those of the same symbols in general formula (R2). 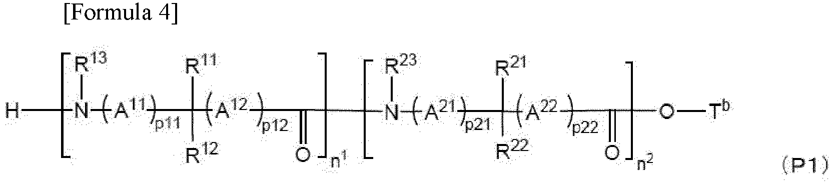
         In formula (P1),
            R¹¹, R¹², R¹³, A¹¹, A¹², p11, p12, and n¹ each represent the same definitions as those of the same symbols in general formula (R1), and
            R²¹, R²², R²³, A²¹, A²², p21, p22, n², and T^{b} each represent the same definitions as those of the same symbols in general formula (R2).

### [Aspect 2]

The method according to Aspect 1, wherein steps (i) and (ii) are carried out continuously as a flow reaction.

### [Aspect 3]

The method according to Aspect 1 or 2, wherein in the reaction of step (i), the compound of formula (R1) and the compound of formula (R2) are used at approximately equal mole ratios.

### [Aspect 4]

The method according to any one of Aspects 1 to 3, wherein the substituent T^{a} of formula (R1) is an aromatic hydrocarbon group having one or more substituents selected from a halogen atom, halogen substitution alkyl group, halogen substitution alkoxy group, nitro group, acetyl group, ester group, sulfonic acid ester group, and amide group.

### [Aspect 5]

The method according to any one of Aspects 1 to 4, wherein the protecting group PG^{a} of formula (R1) is a monovalent hydrocarbon group, acyl group, hydrocarbon oxycarbonyl group, hydrocarbon sulfonyl group, and amide group that may have one or more substituents.

### [Aspect 6]

The method according to any one of Aspects 1 to 5, wherein deprotection of the N-terminal-protected group in formula (S1) at step (ii) is carried out by making the compound of formula (S1) pass through a column filled with basic ion exchange resin.

### [Aspect 7]

The method according to Aspect 6, wherein the basic ion exchange resin is selected from 1,8-diazabicyclo[5.4.0]undec-7-ene (DBU) resin, 1,5-diazabicyclo[4.3.0]non-5-ene (DBN) resin, 1,5,7-triazabicyclo[4.4.0]dec-5-ene (TBD) resin, piperazine resin, dimethylaminopyridine resin, and ammonium resin.

### Effects of the Invention

The method according to the present invention makes it possible to efficiently synthesize a desired peptide chain by continuously elongating the peptide chain via peptide bond reaction.

### BRIEF EXPLANATION OF THE FIGURES

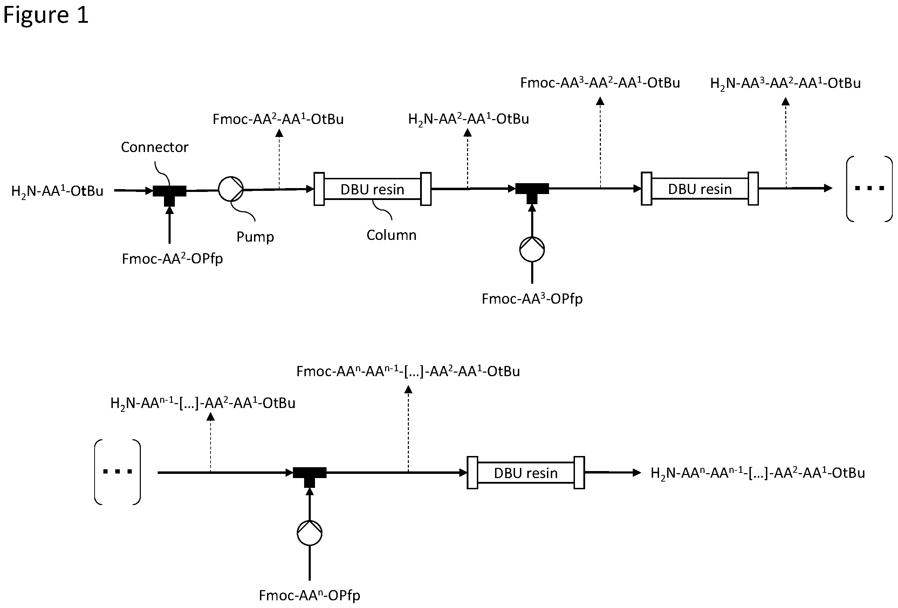

[Figure 1] Figure 1 shows a schematic diagram of an exemplary manufacturing process for synthesizing a polypeptide with the amino acid sequence H₂N-AAⁿ-AAⁿ⁻¹-(...)-AA²-AA¹-O-tBu using the preferred embodiment of the production method of the present invention by flow reaction, where AA¹, AA², ..., AAⁿ⁻¹, and AAⁿ each represent an amino acid residue. In Formula (R1) of this exemplary process, the T^{a} group is a pentafluorophenyl (PFP) group, the PG^{a} group is an Fmoc group, and the T^{b} group in Formula (R2) is a t-butyl (tBu) group.

### EMBODIMENTS

The present invention is described hereinafter in detail with reference to specific embodiments thereof. However, the present invention is not limited to the following embodiments and can be carried out in any embodiment that does not deviate from the gist according to the present invention.

All patent publications, patent application publications, and non-patent documents cited in this disclosure are incorporated herein by reference in their entirety for all purposes.

### [I. Definitions of Terms]

The term "amino acid" herein refers to a compound having a carboxyl group and an amino group. Unless otherwise specified, the type of an amino acid is not particularly limited. For example, from the viewpoint of optical isomerism, an amino acid may be in the D-form, in the L-form, or in a racemic form. From the viewpoint of the relative positions of the carboxyl group and the amino group, an amino acid may be any of an α-amino acid, β-amino acid, γ-amino acid, δ-amino acid, or ε-amino acid. Examples of amino acids include, but are not limited to, natural amino acids that make up proteins. Examples include valine, leucine, isoleucine, alanine, arginine, glutamine, lysine, aspartic acid, glutamic acid, proline, cysteine, threonine, methionine, histidine, phenylalanine, tyrosine, tryptophan, asparagine, glycine, and serine.

The term "peptide" herein refers to a compound comprising a plurality of amino acids linked together via peptide bonds. Unless otherwise specified, the plurality of amino acid units constituting a peptide may be the same type of amino acid unit or may consist of two or more types of amino acid units. The number of amino acids constituting a peptide is not restricted as long as it is two or more. Examples include 2 (also called "dipeptide"), 3 (also called "tripeptide"), 4 (also called "tetrapeptide"), 5 (also called "pentapeptide"), 6, 7, 8, 9, 10, 15, 20, 30, 40, 50, 100, or more. The term "polypeptide" may also be used to refer to tripeptides and longer peptides.

The term "amino group" herein refers to a functional group represented by any formula of -NH₂, -NRH, and -NRR' (where R and R' each represent a substituent) obtained by removing hydrogen from ammonia, a primary amine, and a secondary amine, respectively.

Unless otherwise specified, a hydrocarbon group herein may be either aliphatic or aromatic. An aliphatic hydrocarbon group may be in the form of either a chain or a ring. A chain hydrocarbon group may be linear or branched. A cyclic hydrocarbon group may be monocyclic, bridged cyclic, or spirocyclic. The hydrocarbon group may be saturated or unsaturated. In other words, one, two, or more carbon-carbon double and/or triple bonds may be included. Specifically, "hydrocarbon group" represents a concept including an alkyl group, alkenyl group, alkynyl group, cycloalkyl group, cycloalkenyl group, cycloalkynyl group, aryl group, arylalkyl group, alkylaryl group, etc. Unless otherwise specified, one, two, or more hydrogen atoms of the hydrocarbon group may be replaced with any substituents and one, two, or more carbon atoms in the hydrocarbon group may be replaced with any heteroatoms corresponding to the valence thereof.

The term "hydrocarbon oxy group" herein refers to a group comprising an oxy group (-O-) linked via one bond thereof to the hydrocarbon group as defined above. In other words, the term hydrocarbon oxy group is a concept that includes alkyloxy groups, alkenyloxy groups, alkynyloxy groups, cycloalkyloxy groups, cycloalkenyloxy groups, cycloalkynyloxy groups, aryloxy groups, etc.

The term "hydrocarbon carbonyl group" herein refers to a group comprising a carbonyl group (-C(=O)-) linked via one bond thereof to the hydrocarbon group as defined above. In other words, the term 'hydrocarbon carbonyl group' includes alkyl carbonyl groups, alkenyl carbonyl groups, alkynyl carbonyl groups, cycloalkyl carbonyl groups, cycloalkenyl carbonyl groups, cycloalkynyl carbonyl groups, aryl carbonyl groups, etc.

The term "hydrocarbon sulfonyl group" herein refers to a group comprising a sulfonyl group (-S(=O)₂-) linked via one bond thereof to the hydrocarbon group as defined above. In other words, the term 'hydrocarbon sulfonyl group' includes alkyl sulfonyl groups, alkenyl sulfonyl groups, alkynyl sulfonyl groups, cycloalkyl sulfonyl groups, cycloalkenyl sulfonyl groups, cycloalkynyl sulfonyl groups, aryl sulfonyl groups, etc.

A heterocyclic group may be saturated or unsaturated. In other words, it may contain one, two, or more carbon-carbon double and/or triple bonds. A heterocyclic group may be monocyclic, bridged cyclic, or spirocyclic. The heteroatom included in the constituent atoms of the heterocyclic group is not particularly limited, examples thereof including nitrogen, oxygen, sulfur, phosphorus, and silicon.

The term "heterocyclic oxy group" herein refers to a group comprising an oxy group (-O-) linked via one bond thereof to the heterocyclic group as defined above.

The term "heterocyclic carbonyl group" herein refers to a group comprising a carbonyl group (-C(=O)-) linked via one bond thereof to the heterocyclic group as defined above.

The term "heterocyclic sulfonyl group" herein refers to a group comprising a sulfonyl group (-S(=O)₂-) linked via one bond thereof to the heterocyclic group as defined above.

The term "metalloxy group" (that may have one or more substituents) herein refers to a group represented by formula (R)ₙ-M-O-, where M refers to a metal element, R refers to a substituent, n refers to an integer of 0 or more but 8 or less corresponding to the coordination number of the metal element M.

Unless otherwise specified, the term "substituent" herein refers, independently of each other, to any substituent which is not particularly limited so long as the amidation step of the production method according to the present invention proceeds. Examples include, but are not limited to, a halogen atom, hydroxyl group, carboxyl group, nitro group, cyano group, thiol group, sulfonic acid group, amino group, amide group, imino group, imide group, hydrocarbon group, heterocyclic group, hydrocarbon oxy group, hydrocarbon carbonyl group (acyl group), hydrocarbon oxycarbonyl group, hydrocarbon carbonyloxy group, hydrocarbon substitution amino group, hydrocarbon substitution amino carbonyl group, hydrocarbon carbonyl substitution amino group, hydrocarbon substitution thiol group, hydrocarbon sulfonyl group, hydrocarbon oxysulfonyl group, hydrocarbon sulfonyloxy group, heterocyclic oxy group, heterocyclic carbonyl group, heterocyclic oxycarbonyl group, heterocyclic carbonyloxy group, heterocyclic amino group, heterocyclic amino carbonyl group, heterocyclic carbonyl substitution amino group, heterocyclic substitution thiol group, heterocyclic sulfonyl group, heterocyclic oxysulfonyl group, and heterocyclic sulfonyloxy group. The term "substituents" also may include functional groups obtained by substituting any of the aforementioned functional groups with any of the aforementioned functional groups as long as the valence and physical properties thereof permit. When a functional group has one or more substituents, the number of the substituents is not particularly limited as long as the valence and physical properties thereof permit. When the functional group has two or more substituents, they may be identical to each other or different from each other.

The main abbreviations used herein are listed in Tables 1-1 and 1-2 below.

**[Table 1-1]**

| Abbreviation | Definition |
|---|---|
| Ac | acetyl |
| acac | acetyl acetonate |
| Alloc | allyoxycarbonyl |
| Bn or Bzl | benzyl |
| Boc | tert-butoxycarbonyl |
| Bu | butyl |
| Bz | benzoyl |
| Cbz | benzyloxycarbonyl |
| Cp | cyclopentadienyl |
| 2,4-DNP | 2,4-dinitrophenyl |
| Et | ethyl |
| Fmoc | 9-fluorenylmethyloxycarbonyl |
| HOBt | 1-hydroxybenzotriazole |
| i-Pr | isopropyl |
| MABR | methylaluminum bis(4-bromo-2,6-di-tert-butylphenoxyde) |
| Me | methyl |
| Ms | mesyl |
| Ns | 2 or 4-nitrobenzenesulfonyl |

**[Table 1-2]**

| Abbreviation | Definition |
|---|---|
| Pfp | Pentafluorophenyl |
| Phth | phthaloyl |
| PMB | paramethoxybenzyl |
| PMPCO | paramethoxybenzoyl |
| Pr | propyl |
| TBAF | tetrabutylammonium fluoride |
| TBDPS | tert-butyl diphenylsilyl |
| TBS | tritert-butylsilyl |
| t-Bu | tert-butyl |
| TES | triethylsilyl |
| Tf | trifluoromethane sulfonyl |
| THF | tetrahydrofuran |
| TIPS | triisopropylsilyl |
| TMS | trimethylsilyl |
| TMS-IM | trimethylsilyl imidazole |
| TMS-OTf | trimethylsilyl trifluoromethanesulfonate |
| Troc | 2,2,2-trichloroethoxycarbonyl |
| Trt | trityl |
| Ts | toluenesulfonyl |
| wscHCl | 1-ethyl-3-(3-dimethylaminopropyl)carbodiimide hydrochloride |

Amino acids and residues thereof may herein be represented by three-letter abbreviations well known to a person skilled in the art. The three-letter abbreviations of major amino acids are shown in the following table.

**[Table 2]**

| Abbreviation | Definition |
|---|---|
| Ala | Alanine |
| Arg | Arginine |
| Asn | Asparagine |
| Asp | Aspartic acid |
| Cys | Cysteine |
| Gln | Glutamine |
| Glu | Glutamic acid |
| Gly | Glycine |
| His | Histidine |
| Ile | Isoleucine |
| Leu | Leucine |
| Lys | Lysine |
| Met | Methionine |
| Phe | Phenylalanine |
| Phg | Phenylglycine |
| Pro | Proline |
| Ser | Serine |
| Thr | Threonine |
| Trp | Tryptophan |
| Tyr | Tyrosine |
| Val | Valine |

β-homoamino acids and residues thereof may herein be represented by "Ho" followed by three-letter abbreviations of corresponding β-amino acids.

### [II. Method for producing polypeptide compounds]

### *Summary

An aspect of the present invention relates to a method for producing a polypeptide compound, including at least steps (i) to (iii) below (hereafter also referred to as "the method for producing the peptide compound according to the present invention" or simply as "the production method of the present invention"):
(i) forming an amide bond between the substituted carboxyl group on the right side of an N-terminal-protected amino acid ester or peptide ester compound represented by formula (R1) and the amino group on the left side of an amino acid or peptide or amino acid ester or peptide ester compound represented by formula (R2) to thereby produce an N-terminal-protected peptide compound represented by formula (S1) (amide bond formation reaction, condensation reaction);
(ii) deprotecting the N-terminal of the compound of formula (S1) from step (i) to thereby produce a peptide compound represented by formula (P1) (deprotection reaction); and
(iii) repeating steps (i) and (ii) with using the compound of formula (P1) from step (ii) as a compound of formula (R2) of step (i) to thereby extend the peptide chain via amidation.

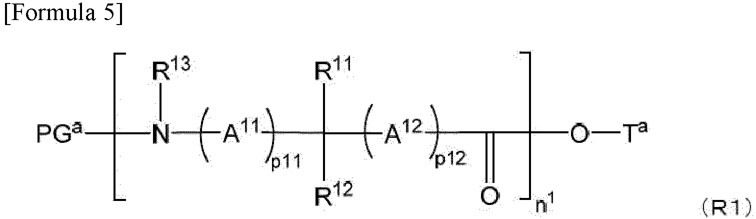

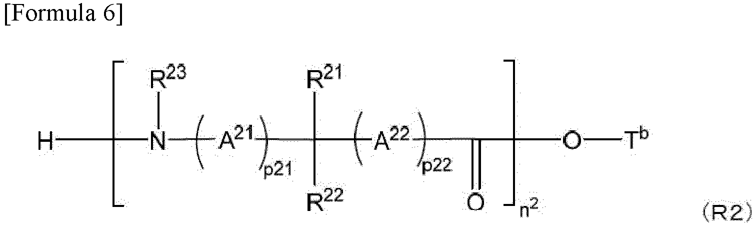

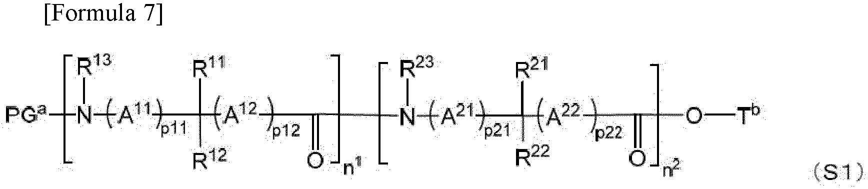

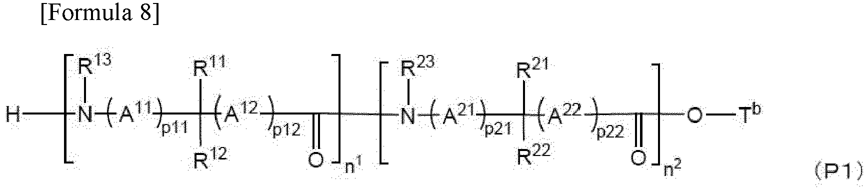

According to the present invention, an N-terminal protected amino acid or peptide ester (R1) with a C-terminal esterified with a monovalent aromatic hydrocarbon group or heterocyclic group T^{a} with an electron-withdrawing substituent is used as an electrophilic compound and mixed with a nucleophilic compound such as an amino acid or peptide or its ester (R2) to cause a peptide bond reaction (condensation reaction) (step (i)), after which the N-terminal of the N-terminal protected amino acid or peptide (S1) obtained by the reaction is deprotected (step (ii)), and the deprotected amino acid or peptide (P1) is then subjected to a peptide bond reaction with another electrophilic compound (R1) (step (iii)). Repeating these steps makes it possible to continuously extend the peptide chain through peptide bond reactions, and it becomes possible to efficiently synthesize the desired peptide chain.

Specifically, the production method of the present invention can dramatically improve the reactivity of an electrophile amino acid by using an N-terminal protected amino acid or peptide ester (R1) with an esterified C-terminus by an aromatic hydrocarbon group or heterocyclic group T^{a} with an electron-withdrawing substituent as an electrophile. This makes it possible to quantitatively progress the reaction even when the molar ratio of electrophilic and nucleophilic amino acids approaches 1:1. This prevents the generation of excess amino acids that do not participate in the reaction and makes it possible to avoid side reactions caused by unreacted substances.

In addition, according to a preferred embodiment of the production method of the present invention, a specific protective group that can be removed by passing through a basic ion exchange resin can be used as the N-terminal protective group PG^{a} of the amino acid or peptide ester (R1) of the electrophile. This makes it possible to carry out the peptide bond reaction (step (i)), in which the N-terminal protected amino acid or peptide ester (R1), as the electrophile, is mixed with the amino acid or peptide (R2), as the nucleophile, and the deprotection of the resulting N-terminal protected amino acid or peptide (S1) using a basic ion exchange resin (step (ii)) as a series of reactions in succession. In addition, by repeating the above process, it is possible to continuously extend the peptide chain through peptide bond reactions using flow reactions, thereby efficiently synthesizing the desired peptide chain.

There have been some reports of methods which use pentafluorophenyl ester of an amino acid (H-AA-O-Pfp) as electrophiles in peptide bond formation reactions (Non-Patent Literature 7: J. Med. Chem., 2001, 44, 3896-3903; Non-Patent Literature 8: Chem. Eur. J., 2019, 25, 15759-15764; Non-Patent Literature 9: Org. Biomol. Chem., 2003, 1, 965-972; Non-Patent Literature 10: J. Org. Chem., 1995, 60, 6, 1733-1740). These methods are known as useful methods. However, there have been no reports in which electrophilic amino acids and nucleophilic amino acids are used at a 1:1 molar ratio, let alone any examples of their use in flow reactions.

### *Substrate Compounds

The symbols in formulae (R1) and (R2) are defined as follows.

R¹¹, R¹², R²¹, and R²² each represent, independently of each other, a hydrogen atom, halogen atom, hydroxyl group, carboxyl group, nitro group, cyano group, or thiol group, or a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

When each of R¹¹, R¹², R²¹, and/or R²² is a monovalent hydrocarbon group or heterocyclic group that may have one or more substituents, a linking group may intervene between the hydrocarbon group or heterocyclic group and the carbon atom to which it binds. The linking group may be, independently of each other, selected from, although is not limited to, the structures listed below (where, in the chemical formulae below, A represents, independently of each other, a monovalent hydrocarbon group or heterocyclic group that may have one or more substituents. When two A's are present in the same group, they may be identical to each other or different from each other.).

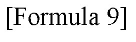

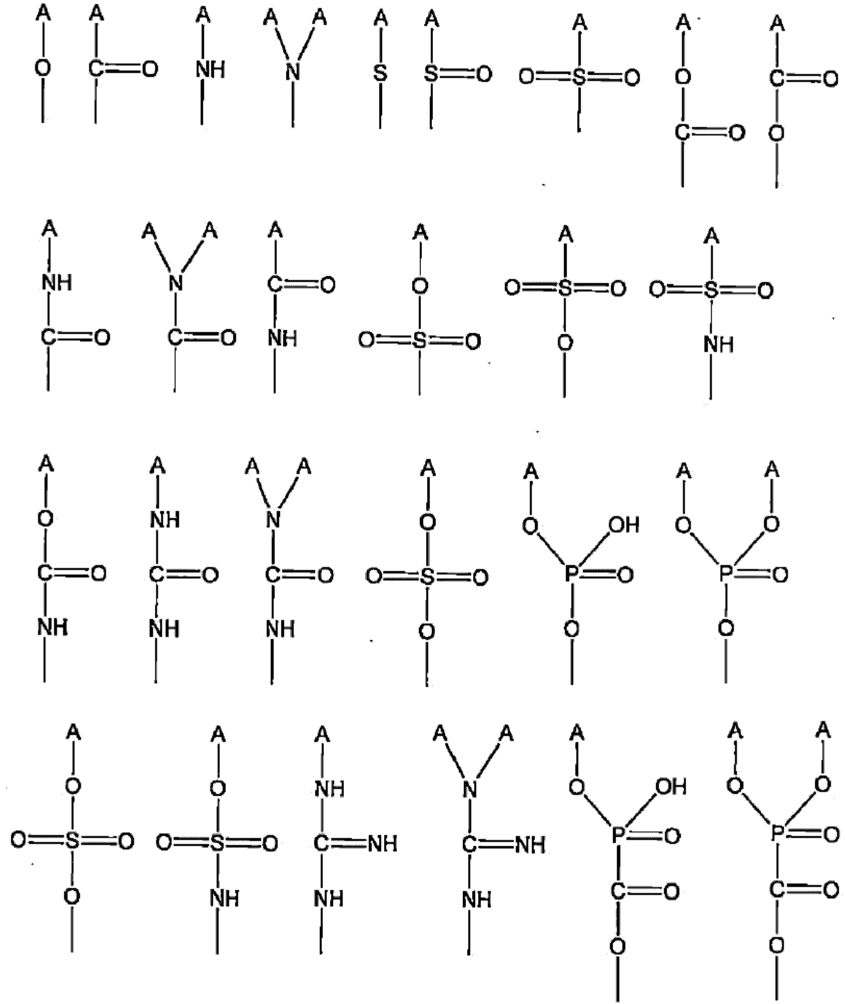

The number of carbon atoms in the hydrocarbon group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The total number of carbon atoms and hetero atoms in the heterocyclic group (when it has one or more substituents, including the number of atoms in the substituents) may be, although is not particularly limited to, the upper limit thereof may be 20 or less, 15 or less, 10 or less, 8 or less, or 6 or less. The lower limit thereof depends on the type of the heterocyclic structure, but may be 3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Among them, each of R¹¹, R¹², R²¹, and R²² may preferably be, independently of each other, a hydrogen atom, hydroxyl group, thiol group, carboxyl group, nitro group, cyano group, or halogen atom, or an amino group, alkyl group, alkenyl group, cycloalkyl group, alkoxy group, aryl group, aryloxy group, acyl group, heterocyclic group, or heterocyclic oxy group that may have one or more substituents.

Specific examples of R¹¹, R¹², R²¹, and R²² may include, although are not limited to, the following.

*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
*Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethylamino group, benzylamino group, and triphenylmethylamino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
*Heterocyclic oxy groups such as furanyloxy group, pyrrolyloxy group, indolyloxy group, and quinolyloxy group.

Of the groups mentioned above, those having a carboxyl group may or may not have a protective group. Although it depends on the reactivity of the compound of formula (R1) and the compound of formula (R2) used in the reaction, if the carboxyl group in any of the above substituents has a protective group, the reaction selectivity with the carboxyl ester group on the right side of the compound represented by formula (R2) may usually be improved over that with the carboxyl group present on the other substituents.

R¹³and R²³ each represent, independently of each other, a hydrogen atom, carboxyl group, or hydroxyl group, or a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

When each of R¹³and/or R²³ is a monovalent hydrocarbon group or heterocyclic group that may have one or more substituents, a linking group may intervene between the hydrocarbon group or heterocyclic group and the nitrogen atom to which it binds. The linking group may be, independently of each other, selected from, although is not limited to, the structures listed below (where, in the chemical formulae below, A represents, independently of each other, a monovalent hydrocarbon group or heterocyclic group that may have one or more substituents. When two A's are present in the same group, they may be identical to each other or different from each other.).

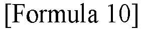

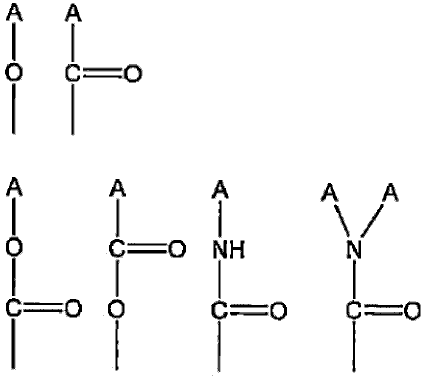

The upper limit for the number of carbon atoms in the hydrocarbon group (when it has one or more substituents, including the number of atoms in the substituents) may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be 3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Each of R¹³and R²³ may preferably be, independently of each other, a hydrogen atom, hydroxyl group, or carboxyl group, or an alkyl group, alkenyl group, cycloalkyl group, alkoxy group, aryl group, aryloxy group, acyl group, heterocyclic group, or heterocyclic oxy group that may have one or more substituents.

Specific examples of R¹³and R²³ may include, although are not limited to, the following.

*Hydrogen atom, hydroxyl group, thiol group, carboxyl group, nitro group, and cyano group;
*Halogen atoms such as fluorine atom, chlorine atom, bromine atom, and iodine atom;
*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
*Alkoxy groups such as methoxy group, ethoxy group, propoxy group, butoxy group, sec-butoxy group, and tert-butoxy group;
* Aryl groups such as phenyl group, benzyl group, tolyl group, naphthyl group, and anthracenyl group;
*Aryloxy groups such as phenyloxy group, benzyloxy group, and naphthyloxy group;
*Acyl groups such as acetyl group, propionyl group, benzoyl group, p-methoxybenzoyl group, and cinnamoyl group;
*Unsubstituted amino group and substitution amino groups such as dimethyl amino group, benzyl amino group, and triphenylmethyl amino group;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, carbazolyl group; and
*Heterocyclic oxy groups such as furanyloxy group, pyrrolyloxy group, indolyloxy group, and quinolyloxy group.

Alternatively, R¹¹ and R¹³ may be bound to each other to form, together with the carbon atom to which R¹¹ binds and the nitrogen atom to which R¹³ binds, a hetero ring that may have one or more substituents, and R²¹ and R²³ may be bound to each other to form, together with the carbon atom to which R²¹ binds and the nitrogen atom to which R²³ binds, a hetero ring that may have one or more substituents. If these groups have one or more substituents, they may be selected arbitrarily from those detailed earlier. The number of substituents may be 5, 4, 3, 2, 1, or 0.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Examples of such hetero rings include, but are not limited to, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, and 2,5-dihydro-1,3-thiazolyl group.

A¹¹, A¹², A²¹, and A²² each represent, independently of each other, a divalent aliphatic hydrocarbon group containing 1 to 3 carbon atoms that may have one or more substituents. Specific Examples include, although are not limited to, methylene group, ethylene group, propylene group, and isopropylene group, as well as groups derived from these groups via substitution with one or more substituents mentioned above. Specific examples of the number of substituents are 3, 2, 1, or 0.

p11, p12, p21, and p22 each represent, independently of each other, 0 or 1.

n¹ represents the number of amino acid units parenthesized with [ ] in formula (R1), and is an integer of 1 or more. When n¹ is 1, the compound of formula (R1) is an amino acid, and when n¹ is 2 or more, the compound of formula (R1) is a peptide. The upper limit for n¹ is not particularly limited so long as the amidation step proceeds, but may preferably be, for example, 100 or less, 80 or less, 60 or less, 50 or less, 40 or less, 30 or less, 20 or less, 15 or less, 12 or less, or 10 or less. Specific examples of n¹ may be 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, or 100.

n² represents the number of amino acid units parenthesized with [ ] in formula (R2), and is an integer of 1 or more. When n² is 1, the compound of formula (R2) is an amino acid, and when n² is 2 or more, the compound of formula (R2) is a peptide. The upper limit for n² is not particularly limited so long as the amidation step proceeds, but may preferably be, for example, 1, 2, 3, 4, 5, 6, 7, 8, 9, or 10.

Needless to say, when n¹ is equal to or greater than 2, then R¹¹, R¹², R¹³, A¹¹, A¹², p11, and p12, which define the structure units parenthesized with [ ], may be either identical to each other or different from each other between the two or more amino acid units. Likewise, when n² is equal to or greater than 2, then R²¹, R²², R²³, A²¹, A²², p21, and p22, which define the structure units parenthesized with [ ], may be either identical to each other or different from each other between the two or more amino acid units. In other words, when each of the compound of formula (R1) and/or the compound of formula (R2) is a peptide, then the two or more amino acid units constituting the peptide may be either identical to each other or different from each other.

In formula (R1), T^{a} represents a monovalent aromatic hydrocarbon group or heterocyclic group having one or more electron-withdrawing substituents. Details of T^{a} will be given later.

In the compound of formula (R2), T^{b} represents a hydrogen atom or a monovalent substituent. Examples of monovalent substituents include, but are not limited to, the groups mentioned above as examples of R¹³ and R²³, as well as protective groups for carboxyl group (hereinafter also referred to as PG^{b}). The protective group PG^{b} for carboxyl group is not restricted, as long as it can be protected so that the carboxyl group does not react in the amidation reaction and can be converted to a carboxyl group by deprotection after the reaction. Details of the protective group PG^{b} for carboxyl group will be given later.

In the compound of formula (R2), the amino group on the left side of the formula may form a salt with a counter acid. In this case, examples of the counter acid include, but are not limited to, aliphatic carboxylic acids with 1 to 5 carbons such as acetic acid and propionic acid; trifluoroacetic acid, hydrochloric acid, hydrobromic acid, sulfuric acid, nitric acid, phosphoric acid, boric acid and sulfonic acid.

Any of the above-mentioned substrate compounds (R1) and (R2) may be used either singly or in combination of any two or more compounds at any ratios.

Some or all of either the substrate compound (R1) or (R2) may be linked or immobilized to a carrier such as a basal plate or resin at any of the substituents. In this case, the type of the carrier such as the basal plate or resin is not limited. Any carriers such as basal plates or resins known so far can be used to the extent that they do not substantially interfere with the amide bond reaction in the production method of the present invention and do not depart from the purpose of the present invention. There is no limitation to the means to link or immobilize the substrate compound to the carrier such as the basal plate or resin, but it may be preferable to form a covalent bond between a substituent of the substrate compound and a substituent present on the basal plate, resin, or other carrier. There are also no limitations on the types of each substituent or the method of forming the covalent bond. Any types of substituents and methods for forming a covalent bond known so far can be used, as long as they do not substantially interfere with the amide bond reaction in the production method of the present invention, and to the extent that they do not depart from the purpose of the present invention. The substrate compound may be linked or immobilized to a basal plate, resin, or other carrier via a covalent bond using a carboxyl or amino group possessed by the substrate compound (other than the carboxyl ester or amino group that is the target of the amide bond reaction formation). Such an embodiment can be regarded similarly to an embodiment in which the carboxyl or amino group possessed by the substrate compound (other than the carboxyl ester or amino group that is the target of the amide bond reaction formation) is protected by introducing a protecting group.

### * C-terminal substituent T^{a} of the electrophilic substrate compound (R1):

In the compound of formula (R1), T^{a} represents a monovalent aromatic hydrocarbon group or heterocyclic group having one or more electron-withdrawing substituents.

Examples of monovalent aromatic hydrocarbon groups include, although are not limited to, phenyl group, benzyl group, tolyl group, naphthyl group, and anthracenyl group. The number of its carbon atoms is not limited, but may be usually 6 or more, and usually 14 or less, or 10 or less.

Examples of monovalent heterocyclic groups include, although are not limited to, furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group. The number of its ring-constituting elements is not limited, but may be usually 5 or more, and usually 14 or less, or 10 or less.

Examples of electron-withdrawing substituents of the monovalent aromatic hydrocarbon group or heterocyclic group include, but are not limited to:
*Halogen atoms (e.g., fluorine, chlorine, bromine, iodine, etc., preferably fluorine or chlorine, etc.);
*Halogen-substituted alkyl groups (e.g., groups derived from the alkyl groups mentioned above via substitution with one or more of the halogen atoms mentioned above);
*Halogen-substituted alkoxy groups (e.g., groups derived from the halogen-substituted alkyl groups mentioned above via linking to an oxy group (-O-));
*Nitro group;
*Acetyl group;
*Carboxyl group (-C(=O)-OH);
*Carboxyl ester groups (e.g., groups derived from the monovalent aliphatic or aromatic hydrocarbon groups that may have one or more substituents (R) mentioned above via linking to a carboxyl group (-C(=O)-OH) (i.e., -C(=O)-O-R));
*Sulfoxy group (-S(=O)₂-OH);
*Sulfoxy ester groups (e.g., groups derived from the monovalent aliphatic or aromatic hydrocarbon group that may have one or more substituents (R) mentioned above via linking to a sulfoxy group (-S(=O)₂-OH) (i.e., -S(=O)₂-O-R));
*Amide group (-C(=O)-NH₂); and
*Substituted amide group (e.g., groups derived from one or more of the monovalent aliphatic or aromatic hydrocarbon groups that may have one or more substituents (R) mentioned above via linking to an amide group (-C(=O)-NH₂) (i.e., -C(=O)-NRH or - C(=O)-NR₂).

The number of the electron-withdrawing substituents that the aromatic hydrocarbon group or heterocyclic group T^{a} have is one or more. The upper limit thereof is not particularly limited, and may be any number that is allowable depending on the type of monovalent aromatic hydrocarbon group or heterocyclic group, examples thereof being 5 or less.

### *Protective group for amino groups:

Various protecting groups for amino groups are known to the art. Examples include monovalent hydrocarbon groups that may have one or more substituents and monovalent heterocyclic groups that may have one or more substituents. Preferred among them include monovalent hydrocarbon groups that may have one or more substituents. However, a linking group may intervene between the hydrocarbon group or heterocyclic group and the nitrogen atom of the amino group it protects. The linking group may be, independently of each other, selected from, although is not limited to, the following groups (where, in the chemical formulae below, A represents, independently of each other, a monovalent hydrocarbon group or heterocyclic group that may have one or more substituents. When two A's are present in the same group, they may be identical to each other or different from each other.).

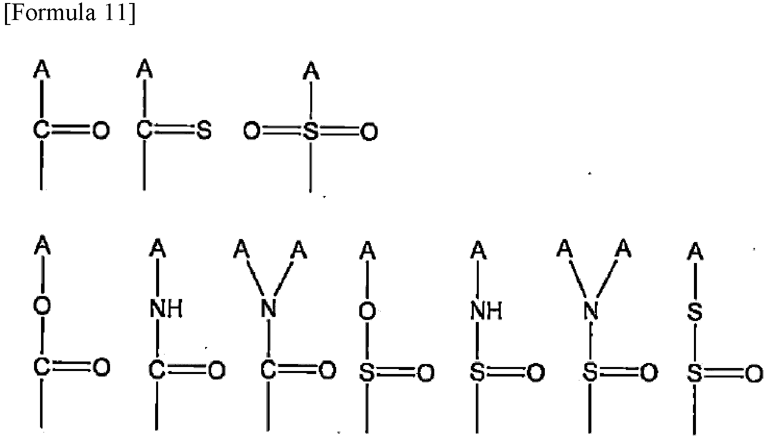

The number of carbons in the protective group may be typically 1 or more, or 3 or more, and typically 20 or less, or 15 or less.

Among them, the amino-protecting group may preferably be one or more selected from the group consisting of monovalent hydrocarbon groups, acyl groups, hydrocarbon oxycarbonyl groups, hydrocarbon sulfonyl groups and amides that may have one or more substituents.

Specific examples of the amino-protective group are listed below. Incidentally, an amino-protective group may be referred to either by the name of the functional group excluding the nitrogen atom of the amino group to which it binds or by the name of the group including the nitrogen atom to which it binds. The following list includes either or both of these names for each protective group.

Examples of unsubstituted or substituted hydrocarbon groups includes: alkyl groups such as methyl group, ethyl group, and propyl group; alkenyl groups such as ethenyl group, propenyl group, and allyl group; alkynyl groups such as propargyl group; cycloalkyl groups such as cyclopropyl group, cyclobutyl group, cyclopentyl group, and cyclohexyl group; aryl groups such as phenyl group, benzyl group, p-methoxybenzyl group, tolyl group, and triphenylmethyl group (Troc group); and substituted hydrocarbon groups such as cyanomethyl group. The number of carbon atoms may typically be 1 or more, or 3 or more, and typically 20 or less, or 15 or less.

Examples of unsubstituted or substituted acyl groups includes: benzoyl group (Bz), o-methoxybenzoyl group, 2,6-dimethoxy benzoyl group, p-methoxybenzoyl group (PMPCO), cinnamoyl group, and phthaloyl group (Phth).

Examples of unsubstituted or substituted hydrocarbon oxycarbonyl groups includes: tert-butoxycarbonyl group (Boc), benzyloxycarbonyl group (Cbz or Z), methoxycarbonyl group, ethoxycarbonyl group, 2-(trimethylsilyl)ethoxycarbonyl group, 2-phenyl ethoxycarbonyl group, 1-(1-adamanthyl)-1-methylethoxycarbonyl group, 1-(3,5-di-t-butylphenyl)-1-methylethoxycarbonyl group, vinyloxycarbonyl group, allyloxycarbonyl group (Alloc), N-hydroxypiperidinyloxycarbonyl group, p-methoxybenzyloxycarbonyl group, p-nitrobenzyloxycarbonyl group, 2-(1,3-dithianyl)methoxycarbonyl, m-nitrophenoxycarbonyl group, 3,5-dimethoxybenzyloxycarbonyl group, o-nitrobenzyloxycarbonyl group, 2,2,2-trichloroethoxycarbonyl group (Troc), and 9-fluorenylmethyloxycarbonyl group (Fmoc).

Examples of unsubstituted or substituted hydrocarbon sulfonyl groups includes:
methane sulfonyl group (Ms), toluenesulfonyl group (Ts), and 2- or 4-nitro benzene sulfonyl (Ns) group.

Examples of unsubstituted or substituted amide groups includes: acetamide, o-(benzoyloxymethyl)benzamide, 2-[(t-butyl-diphenyl-siloxy)methyl]benzamide, 2-toluenesulfonamide, 4-toluenesulfonamide, 2-nitro benzene sulfonamide, 4-nitro benzene sulfonamide, tert-butylsulfinyl amide, 4-toluenesulfonamide, 2-(trimethylsilyl)ethane sulfonamide, and benzyl sulfonamide.

In terms of deprotection methods, the protective group may be deprotected by, e.g., at least one of the following methods: deprotection by hydrogenation, deprotection by weak acid, deprotection by fluorine ion, deprotection by one-electron oxidizing agent, deprotection by hydrazine, and deprotection by oxygen. In particular, according to the production method of the present invention, as the protective group PG^{a} for the terminal amino group of the amino acid or peptide ester (R1) of the electrophile, it is preferable to use a protective group PG^{a} that can be removed by passing it through a basic ion exchange resin.

Preferred examples of the amino protective group include mesyl group (Ms), tert-butoxycarbonyl group (Boc), benzyl group (Bn or Bzl), benzyloxycarbonyl group (Cbz), benzoyl group (Bz), p-methoxybenzyl group (PMB), 2,2,2-trichloroethoxycarbonyl group (Troc), allyloxycarbonyl group (Alloc), 2,4-dinitrophenyl group (2,4-DNP), phthaloyl group (Phth), p-methoxybenzoyl group (PMPCO), cinnamoyl group, toluenesulfonyl group (Ts), 2- or 4-nitrobenzenesulfonyl group (Ns), cyanomethyl group, and 9-fluorenylmethyloxycarbonyl group (Fmoc). These protective groups are preferred because, as mentioned above, they can easily protect the amino group and can be removed under relatively mild conditions.

More preferable examples of the amino protective group include mesyl group (Ms), tert-butoxycarbonyl group (Boc), benzyloxycarbonyl group (Cbz), benzyl group (Bn), p-methoxybenzyl group (PMB), 2,2,2-trichloroethoxycarbonyl group (Troc), allyloxycarbonyl group (Alloc), p-methoxybenzoyl group (PMPCO), benzoyl group (Bz), cyanomethyl group, cinnamoyl group, 2- or 4-nitrobenzenesulfonyl group (Ns), toluenesulfonyl group (Ts), phthaloyl group (Phth), 2,4-dinitrophenyl group (2,4-DNP), and 9-fluorenylmethyloxycarbonyl group (Fmoc).

As mentioned above, according to the production method of the present invention, as the protective group PG^{a} for the terminal amino group of the amino acid or peptide ester (R1) of the electrophile, it is preferable to use a protective group PG^{a} that can be removed by passing it through a basic ion exchange resin. Examples thereof include: 9-fluorenyl methyloxycarbonyl group (Fmoc group), benzyloxycarbonyl group (Cbz group), tert-butoxycarbonyl group (Boc group), ally oxycarbonyl group (Alloc group), and p-methoxy benzyl group (PMB group). Preferred among these is Fmoc group.

### *Protective group for carboxyl groups:

Various protective groups for carboxyl groups are known to the art. Examples include a monovalent hydrocarbon group or heterocyclic group that may have one or more substituents. If these groups have one or more substituents, they may be selected arbitrarily from those detailed earlier. The number of substituents may be 5, 4, 3, 2, 1, or 0.

The upper limit for the number of carbon atoms in the hydrocarbon group (when it has one or more substituents, including the number of atoms in the substituents) may be 20 or less, 15 or less, 10 or less, 8 or less, or 6 or less. The lower limit thereof depends on the type of the hydrocarbon group, but may be 1 or more in the case of alkyl groups, 2 or more in the case of alkenyl groups or alkynyl groups, and 3 or more, for example 4 or more, or 5 or more in the case of cycloalkyl groups. Specific examples of the number of atoms include 1, 2, 3, 4, 5, 6, 7, 8, 9, 10, 11, 12, 13, 14, 15, 16, 17, 18, 19, or 20.

The upper limit for the total number of carbon atoms and hetero atoms in the heterocyclic group (when it has one or more substituents, including the number of atoms in the substituents) may be 20 or less, 15 or less, 10 or less, 8 or less, or 6 or less. The lower limit thereof depends on the type of the heterocyclic structure, but may be3 or more, for example 4 or more, or 5 or more. Specific examples of the number of atoms include 3, 4, 5, 6, 7, 8, 9, 10, 11, 12, 13, 14, 15, 16, 17, 18, 19, or 20.

Specific examples of protective group for carboxyl groups may include, although are not limited to, the following.

*Alkyl groups such as methyl group, ethyl group, propyl group, isopropyl group, n-butyl group, isobutyl group, tert-butyl group, sec-butyl group, pentyl group, isopentyl group, neopentyl group, hexyl group, heptyl group, octyl group, decyl group, and nonyl group;
*Alkenyl groups such as ethenyl group, propenyl group, allyl group, butenyl group, pentenyl group, hexenyl group, heptenyl group, and octenyl group;
*Alkynyl groups such as propargyl group;
*Cycloalkyl groups such as cyclopropyl group, cyclobutyl group, cyclopentyl group, cyclohexyl group, cycloheptyl group, bicyclooctyl group, and spirooctyl group;
* Aryl groups such as phenyl, benzyl, tolyl, naphthyl and anthracenyl groups;
*Heterocyclic groups such as furanyl group, thiophenyl group, pyranyl group, pyrrolinyl group, pyrrolyl group, 2,3-dihydro-1H-pyrrolyl group, piperidinyl group, piperazinyl group, homopiperazinyl group, morpholino group, thiomorpholino group, 1,2,4,6-tetrahydro pyridyl group, hexahydro pyrimidyl group, hexahydro pyridazyl group, 1,2,4,6-tetrahydro pyridyl group, 1,2,4,6-tetrahydro pyridazyl group, 3,4-dihydropyridyl group, imidazolyl group, 4,5-dihydro-1H-imidazolyl group, 2,3-dihydro-1H-imidazolyl group, pyrazolyl group, 4,5-dihydro-1H-pyrazolyl group, 2,3-dihydro-1H-pyrazolyl group, oxazolyl group, 4,5-dihydro-1,3-oxazolyl group, 2,3-dihydro-1,3-oxazolyl group, 2,5-dihydro-1,3-oxazolyl group, thiazolyl group, 4,5-dihydro-1,3-thiazolyl group, 2,3-dihydro-1,3-thiazolyl group, 2,5-dihydro-1,3-thiazolyl group, and carbazolyl group; and
*Silicon-based protective groups such as trimethylsilyl (TMS) group, triethylsilyl (TES) group, triisopropylsilyl (TIPS) group, tri(tert-butyl)silyl (TBS) group, tert-butyldiphenylsilyl (TBDPS) group and tris(trialkylsilyl)silyl group.

### *Silane compounds:

In the production method (1) of the present invention, a silane compound may coexist in the reaction system. Carrying out the reaction with a silane compound in the reaction system may result in various advantages such as improved reaction yield and stereoselectivity.

Examples of silane compounds include: various tris{halo-(preferably fluorine-)substituted alkyl }silanes such as HSi(OCH(CF₃)₂)₃, HSi(OCH₂CF₃)₃, HSi(OCH₂CF₂CF₂H)₃, HSi(OCH₂CF₂CF₂CF₂CF₂H)₃; as well as trimethylsilyl trifluoromethanesulfonate(TMS-OTf), 1-(trimethylsilyl)imidazole (TMSIM), dimethyl ethylsilyl imidazole (DMESI), dimethyl isopropylsilyl imidazole (DMIPSI), 1-(tert-butyl dimethylsilyl)imidazole (TBSIM), 1-(trimethylsilyl)triazole, 1-(tert-butyl dimethylsilyl)triazole, dimethylsilyl imidazole, dimethylsilyl (2-methyl)imidazole, trimethyl bromosilane (TMBS), trimethyl chlorosilane (TMCS), N-methyl-Ntrimethylsilyl trifluoroacetamide (MSTFA), N,O-bis(trimethylsilyl)trifluoroacetamide (BSTFA), N,O-bis(trimethylsilyl)acetamide (BSA), N-(trimethylsilyl)dimethyl amine (TMSDMA), N-(tert-butyl dimethylsilyl)-N-methyl trifluoroacetamide (MTBSTFA), and hexamethyldisilazane (HMDS). Any one of these may be used alone, or two or more may be used together in any combination and ratio.

However, when the production method of the present invention is carried out in a flow reaction, it is preferable not to use such silane compounds in order to omit processes such as the removal of unnecessary components after the reaction and to improve efficiency.

### *Lewis acid catalyst:

In the production method of the present invention, the reaction system may also contain a Lewis acid catalyst. Carrying out the reaction with a Lewis acid catalyst in the reaction system may lead to various advantages, such as improved reaction yield and stereoselectivity. On the other hand, however, when a Lewis acid catalyst is used, it may be necessary to separate and remove the Lewis acid catalyst from the reaction product. Therefore, it is preferable to determine whether to use a Lewis acid catalyst taking into consideration the purpose of using the production method according to the present invention.

When a Lewis acid catalyst is used in the production method of the present invention, the type of catalyst is not limited, but it may preferably be a metal compound that functions as a Lewis acid. Examples of metal elements constituting the metal compound include various metals belonging to groups 2 through 15 of the periodic table. Examples of such metal elements include boron, magnesium, gallium, indium, silicon, calcium, lead, bismuth, mercury, transition metals, and lanthanoid elements. Examples of transition metals include scandium, titanium, vanadium, chromium, manganese, iron, cobalt, nickel, copper, zinc, yttrium, zirconium, niobium, molybdenum, technetium, ruthenium, rhodium, palladium, tin, silver, cadmium, hafnium, tantalum, tungsten, rhenium, osmium, iridium, platinum, gold, and thallium. Examples of lanthanoid elements include lanthanum, cerium, neodymium, samarium, europium, gadolinium, holmium, erbium, thulium, ytterbium. From the viewpoint of excellent reaction acceleration and highly stereoselective production of amide compounds, the metal element may preferably be one or more selected from titanium, zirconium, hafnium, tantalum, niobium, boron, vanadium, tungsten, neodymium, iron, lead, cobalt, copper, silver, palladium, tin, and thallium, more preferably one or more selected from titanium, zirconium, hafnium, tantalum, and niobium. The metal compound may contain one, two or more metal atoms. If the metal compound contains two or more metal atoms, the two or more metal atoms may be either of the same metal element or of different metal elements.

Ligands constituting the metal compound may be selected according to the type of the metal element. Examples of ligands include: substituted or unsubstituted linear- or branched-chain alkoxy groups containing 1 to 10 carbon atoms, such as methoxy group, ethoxy group, propoxy group, butoxy group, trifluoroethoxy group, and trichloroethoxy group; halogen atoms such as fluorine atom, chlorine atom, bromine atom, iodine atom; aryloxy groups having 1 to 10 carbon atoms; acetylacetonate group (acac), acetoxy group (AcO), trifluoromethane sulfonate group (TfO); substituted or unsubstituted linear- or branched-chain alkyl groups having 1 to 10 carbon atoms; phenyl group, oxygen atom, sulfur atom, group -SR (where R represents a substituent exemplified by substituted or unsubstituted hydrocarbon groups containing 1 to 20 carbon atoms), group -NRR' (where R and R', independently of each other, represent a hydrogen atom or a substituent exemplified by substituted or unsubstituted hydrocarbon groups containing 1 to 20 carbon atoms), and cyclopentadienyl (Cp) group.

Preferred metal compounds among these are titanium compounds, zirconium compounds, hafnium compounds, tantalum compounds, or niobium compounds. Examples of these metal compounds are indicated below. Any one of these may be used alone, or two or more may be used together in any combination and ratio.

Examples of titanium compounds include those represented by TiX¹₄ (where 4 X¹'s, independently of each other, represent any of the ligands exemplified above, provided that 4 X¹'s may be the same type of ligand or different from each other.). When X¹ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X¹ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X¹ is a halogen atom, it may preferably be a chlorine atom or a bromine atom. Preferred examples of titanium compounds include Ti(OMe)₄, Ti(OEt)₄, Ti(OPr)₄, Ti(Oi-Pr)₄, Ti(OBu)₄, Ti(Ot-Bu)₄, Ti(OCH₂CH(Et)Bu)₄, CpTiCl₃, Cp₂TiCl₂, Cp₂Ti(OTf)₂, (i-PrO)₂TiCl₂, and (i-PrO)₃TiCl.

Examples of zirconium compounds include those represented by ZrX²₄ (where 4 X²'s, independently of each other, represent any of the ligands exemplified above, provided that 4 X²'s may be the same type of ligand or different from each other.). When X² is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X² is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X² is a halogen atom, it may preferably be a chlorine atom or a bromine atom. Preferred examples of zirconium compounds include Zr(OMe)₄, Zr(OEt)₄, Zr(OPr)₄, Zr(Oi-Pr)₄, Zr(OBu)₄, Zr(Ot-Bu)₄, Zr(OCH₂CH(Et)Bu)₄, CpZrCl₃, Cp₂ZrCl₂, Cp₂Zr(OTf)₂, (i-PrO)₂ZrCl₂, and (i-PrO)₃ZrCl.

Examples of hafnium compounds include those represented by HfX³₄ (where 4 X³'s, independently of each other, represent any of the ligands exemplified above, provided that 4 X³'s may be the same type of ligand or different from each other.). When X³ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X³ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X³ is a halogen atom, it may preferably be a chlorine atom or a bromine atom. Preferred examples of hafnium compounds include HfCp₂Cl₂, HfCpCl₃, and HfCl₄.

Examples of tantalum compounds include those represented by TaX⁴₅ (where 5 X⁴'s, independently of each other, represent any of the ligands exemplified above, provided that 5 X⁴'s may be the same type of ligand or different from each other.). When X⁴ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁴ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁴ is a halogen atom, it may preferably be a chlorine atom or a bromine atom. Preferred examples of tantalum compounds include tantalum alkoxide compounds (e.g., compounds in which X⁴ is an alkoxy group) such as Ta(OMe)₅, Ta(OEt)₅, Ta(OBu)s, Ta(NMe₂)₅, Ta(acac)(OEt)₄, TaCl₅, TaCl₄(THF), and TaBr₅. Other examples are those in which X⁴ is an oxygen, such as Ta₂O₅.

Examples of niobium compounds include those represented by NbX⁵₅ (where 5 X⁵'s, independently of each other, represent any of the ligands exemplified above, provided that 5 X⁵'s may be the same type of ligand or different from each other.). When X⁵ is an alkoxy group, it may preferably be a linear- or branched-chain alkoxy group having 1 to 10 carbon atoms, more preferably a linear- or branched-chain alkoxy group having 1 to 5 carbon atoms, still more preferably a linear- or branched-chain alkoxy group having 1 to 4 carbon atoms. When X⁵ is an aryloxy group, it may preferably be an aryloxy group having 1 to 20 carbon atoms, more preferably an aryloxy group having 1 to 15 carbon atoms, still more preferably an aryloxy group having 1 to 10 carbon atoms. These ligands may have one or more substituents. When X⁵ is a halogen atom, it may preferably be a chlorine atom or a bromine atom. Preferred examples of niobium compounds include niobium alkoxide compounds (e.g., compounds in which X⁵ is an alkoxy group) such as NbCl₄(THF), NbCl₅, Nb(OMe)₅, and Nb(OEt)₅. Other examples are those in which X⁵ is an oxygen, such as Nb₂O₅.

The Lewis acid catalyst may be loaded on a carrier. There are no particular restrictions on the carrier on which the Lewis acid catalyst is to be loaded, and any known carrier can be used. Also, any known method can be used to load the Lewis acid catalyst on the carrier.

However, when the production method of the present invention is carried out in a flow reaction, it is preferable not to use such Lewis acid catalysts in order to omit processes such as the removal of unnecessary components after the reaction and to improve efficiency.

### *Other ingredients:

In the production method of the present invention, any other ingredients may coexist in the reaction system. Examples of other ingredients may include, although are not limited to, conventional catalysts (other than Lewis acid catalysts) that can be used for an amidation reaction, bases, phosphorus compounds, and solvents. Any one of these may be used alone, or two or more may be used together in any combination and ratio.

Examples of catalysts (other than Lewis acid catalysts) include methylaluminum bis(4-bromo-2,6-di-tert-butylphenoxyde (MABR), trimethylsilyl trifluoromethanesulfonate (TMS-OTf), and methylaluminum bis(2,6-di-tert-butylphenoxyde (MAD). Any one of these may be used alone, or two or more may be used together in any combination and ratio.

The type of base is not restricted, and any base that is known to improve reaction efficiency can be used. Examples of such bases include amines having 1 to 4 linear or branched-chain alkyl groups with 1 to 10 carbons, such as tetrabutylammonium fluoride (TBAF), triethylamine (Et₃N), diisopropylamine (i-Pr₂NH), and diisopropylethylamine (i-Pr₂EtN), as well as inorganic bases such as cesium fluoride. Any one of these may be used alone, or two or more may be used together in any combination and ratio.

Examples of phosphorus compounds include: phosphine compounds such as trimethyl phosphine, triethyl phosphine, tripropyl phosphine, trimethyloxyphosphine, triethyloxyphosphine, tripropyloxyphosphine, triphenyl phosphine, trinaphthyl phosphine, triphenyloxyphosphine, tris(4-methylphenyl)phosphine, tris(4-methoxy phenyl)phosphine, tris(4-fluorophenyl)phosphine, tris(4-methylphenyloxy)phosphine, tris(4-methoxy phenyloxy)phosphine, and tris(4-fluorophenyloxy)phosphine; phosphate compounds such as trimethyl phosphate, triethyl phosphate, tripropyl phosphate, trimethyloxyphosphate, triethyloxyphosphate, tripropyloxyphosphate, triphenyl phosphate, trinaphthyl phosphate, triphenyloxyphosphate, tris(4-methylphenyl)phosphate, tris(4-methoxy phenyl)phosphate, tris(4-fluorophenyl)phosphate, tris(4-methylphenyloxy)phosphate, tris(4-methoxy phenyloxy)phosphate, and tris(4-fluorophenyloxy)phosphate; multivalent phosphine compounds or multivalent phosphate compounds such as 2,2'-bis(diphenylphosphino)-1,1'-binaphthyl (BINAP) and 5,5'-bis(diphenylphosphino)-4,4'-bi-1,3-benzodioxole (SEGPHOS). Any one of these may be used alone, or two or more may be used together in any combination and ratio.

However, when the production method of the present invention is carried out in a flow reaction, it is preferable not to use such other ingredients in order to omit processes such as the removal of unnecessary components after the reaction and to improve efficiency.

### *Reaction solvent:

From the viewpoint of increasing reaction efficiency, the reaction may be carried out in a solvent. In particular, when the production method of the present invention is carried out in a flow reaction, a solvent is to be used.

Examples of solvents include, although are not limited to, aqueous solvents and organic solvents. Examples of organic solvents may include, although are not limited to: aromatic hydrocarbons such as toluene and xylene; pentane; ethers such as petroleum ether, tetrahydrofuran (THF), 1-methyl tetrahydrofuran (1-MeTHF), diisopropyl ether (i-Pr₂O), diethyl ether (Et₂O), and cyclopentylmethyl ether (CPME); nitrogen-containing organic solvents acetonitrile (MeCN); chlorine-containing organic solvents such as dichloromethane (DCM); esters such as ethyl acetate (AcOEt); and organic acids such as acetic acids. Any one of these solvents may be used alone, or two or more may be used together in any combination and ratio.

The solvent used in the amide bond formation reaction (step (i)) and the solvent used in the deprotection (step (ii)) may be the same or different. If different solvents are used, the solvent used in the amide bond formation reaction (step (i)) must be replaced before the product is subjected to the deprotection (step (ii)). Therefore, when the production method of the present invention is carried out in a flow reaction, it is preferable, from the perspective of efficiency, to use the same solvent for the amide bond formation reaction (step (i)) and the deprotection (step (ii)). Examples of solvents that can be used for both the amide bond formation reaction (step (i)) and deprotection (step (ii)) include THF, dichloromethane, chloroform, acetonitrile, and CPME, of which THF is the most preferable.

### *Amide bond forming reaction:

According to the production method of the present invention, an amide bond is formed between the aforementioned electrophilic substrate, i.e., the N-terminal protected amino acid or peptide ester (R1), and the nucleophilic substrate, i.e., the amino acid or peptide or its ester (R2), to produce an N-terminal protected peptide (S1) (step (i)). The procedure and conditions for such a reaction are not limited, but are preferably as follows.

The amount ratio between the electrophilic substrate compound (R1) and the nucleophilic substrate compound (R2) is not restricted, but relative to 1 mol of the electrophilic substrate compound (R1), the nucleophilic substrate compound (R2) may be used in an amount within a range of 0.1 mol or more, or 0.2 mol or more, or 0.3 mol or more, or 0.4 mol or more, or 0.5 mol or more, and 20 mol or less, or 10 mol or less, or 5 mol or less, or 4 mol or less, or 3 mol or less. Needless to say, it is necessary to use at least 1 mol each of the substrate compounds (R1) and (R2) for the resulting compound (S1) to be manufactured.

Specifically, when the production method of the present invention is carried out in a flow reaction, it is preferable to use the substrate compounds (R1) and (R2) in a ratio as close as possible to the stoichiometric ratio. More specifically, in the step of synthesizing a dipeptide using amino acids as both the substrate compounds (R1) and (R2), relative to 1 mol of either substrate compound, the amount of the other substrate compound may preferably be kept within a range of, e.g., 1.5 moles or less, 1.4 moles or less, 1.3 moles or less, 1.2 moles or less, 1.1 moles or less, or even 1.05 moles or less, for every 1 mole of the other substrate compound. or 1.4 moles or less, or 1.3 moles or less, or 1.2 moles or less, or 1.1 moles or less, or even 1.05 moles or less. On the other hand, when using an amino acid as the substrate compound (R1) and a peptide consisting of two or more amino acids as the substrate compound (R2) to synthesize peptides consisting of three or more amino acids, then the substrate amino acid (R1) always functions as an electrophilic amino acid, so even if unreacted electrophilic amino acid molecules are deprotected, they are thought to be immediately converted into inactive diketopiperazines through self-condensation. Accordingly, even if a slightly excessive amount of the substrate amino acid (R1) is added, it is thought that there will be little adverse effect on future reactions. Therefore, in this case, relative to 1 mol of either substrate compound, the other substrate compound may preferably be used in a range of, for example, 3 moles or less, or 2.5 moles or less, or 2.0 moles or less, or 1.5 moles or less.

When a silane compound is used, the amount used is not restricted, but when the amount of the compound of formula (R1) is 100 mol %, the silane compound may be used in an amount of 0.1 mol % or more, or 0.2 mol % or more, or 0.3 mol % or more, or 50 mol % or less, or 30 mol % or less, or 2 0 mol % or less, or 15 mol % or less.

When a Lewis acid catalyst is used, the amount used is not restricted, but when the amount of the compound of formula (R1) is 100 mol%, the Lewis acid catalyst may be used in an amount of 0.1 mol% or more, or 0.2 mol% or more, or 0.3 mol% or more, or 50 mol% or less, or 30 mol% or less, or 20 mol% or less, or 15 mol% or less.

When other optional ingredients are used, their amounts should be adjusted accordingly, referring, for example, to the previous findings of the inventor and others in previous patents (Patent Literatures 1 to 8).

For all of the above components, it may be possible to add the entire amount at once, to add it in several instalments, or to add it in small amounts continuously. In particular, in the case of flow reactions, it is sufficient to continuously supply solutions containing the substrate compounds (R1) and (R2) in the reaction solvent using a feed pump or similar device, and then allow them to react while circulating through the reaction pathway.

The reaction temperature is not restricted as long as the reaction proceeds, but may be 0 °C or more, or 10 °C or more, or 20 °C or more, and 100 °C or less, or 80 °C or less, or 60 °C or less.

The reaction pressure is not restricted as long as the reaction proceeds, and the reaction may be carried out under reduced, normal, or pressurized pressure, but may typically be carried out under normal pressure.

The reaction atmosphere is also not limited as long as the reaction proceeds, but the reaction may be carried out under an atmosphere of inert gas such as argon or nitrogen.

In the case of flow reactions, the flow rate of each solution of the substrate compounds (R1) and (R2) is not restricted as long as the reaction progresses, but from the perspective of ensuring that the reaction progresses sufficiently and efficiently, it may be 0.01 mL/min or 0.05 mL/min or more, or 0.1 mL/min or more, or, for example, 100 mL/min or less, or 50 mL/min or less, or 20 mL/min or less.

The reaction time is not limited as long as the reaction progresses, but from the perspective of ensuring that the reaction progresses sufficiently, it can be set to, for example, 5 minutes or more, 10 minutes or more, 20 minutes or more, or 30 minutes or more. There is no particular limit on the upper limit, but from the perspective of efficiency, for example, it can be set to within 50 hours, within 20 hours, within 5 hours, or within 1 hour. In the case of flow reactions, in order to ensure that the reaction progresses sufficiently and efficiently, it is sufficient to adjust the time that the solutions of the substrate compounds (R1) and (R2) are in contact within the reaction pathway to within the aforementioned range.

### *Deprotection of the N-terminal-protected peptide:

In the production method of the present invention, the N-terminal protected peptide (S1) obtained by the above-mentioned reaction is subjected to deprotection of its N-terminus to obtain the peptide compound represented by the following formula (P1) (step (ii)). The method of deprotection is not particularly restricted, and it is sufficient to select an appropriate method according to the type of protective group PG^{a}. The method of deprotection is as described above.

Specifically, in the production method of the present invention, it is preferable to carry out this deprotection by passing the compound of formula (S1) through a column packed with basic ion exchange resin. By carrying out the deprotection in this manner, it becomes possible to carry out the deprotection (step (ii)) following the amide bond formation reaction (step (i)) in a flow reaction, and in turn, it becomes possible to carry out the entire production method of the present invention in a flow.

When the N-terminal deprotection of the compound of Formula (S1) is carried out using a column packed with basic ion exchange resin, there are no particular restrictions on the type of the basic ion exchange resin to be used, which can be selected as appropriate according to the type of the protective group PG^{a} and the production conditions, etc. Examples include 1,8-diazabicyclo[5.4.0]undec-7-ene (DBU) resin, 1,5-diazabicyclo[4.3.0]non-5-ene (DBN) resin, 1,5,7-triazabicyclo[4.4.0]dec-5-ene (TBD) resin, piperazine resin, dimethylaminopyridine resin, and ammonium resin.

There are no particular restrictions on the procedure for deprotection, and it is sufficient to pass the compound of formula (S1) obtained in the amide bond formation reaction (step (i)) through a column packed with basic ion exchange resin. In particular, in the case of flow reactions, the reaction solution containing the compound of formula (S1) obtained in the above-mentioned amide bond formation reaction (step (i)) can be continuously pumped through a pump or other device and passed through a column packed with basic ion exchange resin.

The temperature of the column during deprotection is not limited as long as deprotection progresses, but can be set, for example, to 0°C or above, 10°C or above, or 20°C or above, or, for example, to 100°C or below, 80°C or below, or 60°C or below.

The pressure during deprotection is not restricted as long as deprotection progresses, and it can be carried out under reduced pressure, normal pressure, or pressurized conditions, but it may usually be carried out under normal pressure.

In the case of flow reactions, the flow rate of the reaction solution containing the compound of formula (S1) is not restricted as long as the deprotection proceeds, but from the perspective of ensuring that the deprotection proceeds sufficiently and efficiently, it may preferably be 0.01 mL/min or more, or 0.05 mL/min or more, or 0.1 mL/min or more, and for example 100 mL/min or less, or 50 mL/min or less, or 20 mL/min or less.

The time for deprotection is not limited as long as deprotection progresses, but from the perspective of ensuring sufficient and efficient deprotection, it may be set to 10 minutes or more, 20 minutes or more, or 30 minutes or more, and 80 hours or less, 60 hours or less, or 50 hours or less. In the case of flow reactions, the time for deprotection can be adjusted to the above range by adjusting the flow rate of the reaction solution containing the compound of formula (S1) and the length of the column filled with basic ion exchange resin.

### *Elongation of the peptide chain through repeated reactions and deprotection

In the production method of the present invention, the compound of formula (P1) obtained in the deprotection (step (ii)) is used as the nucleophilic substrate compound of formula (R2), and the amide bond formation reaction (step (i)) and the deprotection (step (ii)) are repeatedly carried out (step (iii)). In this manner, the desired amino acids are linked together in sequence by amide bonds to extend the peptide chain, and the desired peptide compound (P1) can be produced. In particular, by repeating these steps while selecting appropriate compounds as substrate compounds (R1) and (R2), it is theoretically possible to synthesize polypeptides with any number of amino acid residues and amino acid sequences.

For example, using the preferred embodiment of the manufacturing method of the present invention by flow reaction, to synthesise a polypeptide with a sequence of H₂N-AAⁿ-AAⁿ⁻¹-(...)-AA²-AA¹-O-tBu, where n polypeptide (where AA¹, AA², ..., AAⁿ⁻¹, and AAn represent amino acid residues, respectively), the following procedure should be used (see the schematic diagram in Figure 1). First, use the N-terminal protected amino acid ester (e.g. Fmoc-AA²-Ot-OPfp) corresponding to AA² as the electrophilic substrate compound (R1), and the amino acid ester (H₂N-AA1-O-tBu) as the nucleophilic substrate compound (R2), and carry out the amide bond formation reaction in step (i) to produce the N-terminal protected dipeptide (Fmoc-AA²-AA¹-O-tBu) of formula (S1). This compound of formula (S1) is then passed through ion exchange resin to carry out the N-terminal deprotection in step (ii) to thereby produce the dipeptide (H₂N-AA²-AA¹-O-tBu) of formula (P1) with an unmodified N-terminal. This dipeptide of formula (P1) is then used as a new nucleophilic substrate compound (R2), and the amide bond formation reaction in step (i) and the deprotection in step (ii) are carried out using the N-terminal protected amino acid ester of formula (R1) corresponding to the next amino acid residue AA³. By repeating this procedure to extend the amino acid residues on the N-terminal side of the peptide chain, the desired polypeptide H₂N-AAⁿ-AAⁿ⁻¹-(...)-AA²-AA¹-O-tBu can be synthesized.

### *Post-treatments (e.g., purification and recovery):

The peptide compound (P1) produced by the production method (1) of the present invention may be subjected to various post-treatments. For example, the peptide compound (P1) produced can be isolated and purified according to conventional methods such as column chromatography and recrystallization. In addition, when the peptide compound (P1) produced has an amino group and/or a carboxyl group each protected by a protective group, deprotection can be performed according to the method described below.

### [III. Others]

The production method of the present invention may be carried out by the sequential method (batch method) or by the continuous method (flow method). The specific details of the sequential method (batch method) and the continuous method (flow method) are well known in the art. However, as described in detail earlier, the production method of the present invention can be carried out by the continuous method (flow method), and this is preferable because it provides various advantages.

The peptide compound (P1) obtained using the production method of the present invention may be subjected to various further post-processing.

For example, the peptide compound (P1) obtained using the production method described above may be isolated and purified using conventional methods such as column chromatography and recrystallisation.

In addition, the peptide compound of formula (P1) obtained by the production method mentioned above can be subjected to deprotection of the amino group protected by the protecting group. The method of deprotecting the protected amino group is not particularly restricted, and various methods can be used depending on the type of the protecting group. Examples include deprotection by hydrogenation, deprotection by weak acids, deprotection by fluorine ions, deprotection by one-electron oxidants, deprotection by hydrazine, and deprotection by oxygen. The deprotection by hydrogenation may be carried out by, e.g.; (a) a method of causing deprotection in the presence of hydrogen gas using a metal catalyst such as palladium, palladium-carbon, palladium hydroxide, palladium-carbon hydroxide, etc., as a reduction catalyst; and (b) a method of causing deprotection in the presence of a metal catalyst such as palladium, palladium-carbon, palladium hydroxide, palladium-carbon hydroxide, etc., using a hydrotreating reductant such as sodium borohydride, lithium aluminum hydride, lithium borohydride, diborane, etc.

Likewise, the peptide compound of formula (P1) obtained by the production method mentioned above can be subjected to deprotection of the carboxyl group protected by the protecting group. The method of deprotecting the protected carboxyl group is not particularly restricted, and various methods can be used depending on the type of the protecting group. Examples include deprotection by hydrogenation, deprotection by bases, and deprotection by weak acids. In the case of deprotection with a base, a strong base such as lithium hydroxide, sodium hydroxide, potassium hydroxide, etc. can be used.

The present inventors have filed the following prior patent applications relating to amidation reactions for linking amino acids or peptides and methods for producing polypeptides thereby. It is possible to perform the various polypeptide production methods of the present invention in combination with the amidation reactions and polypeptide production methods described in these earlier patent applications as appropriate and/or to modify the amidation reactions and polypeptide production methods described in these prior patent applications as appropriate, taking into account the conditions of these prior patent applications. The descriptions of these earlier patent applications are incorporated herein by reference in their entirety.
(1) WO 2017/204144 A (Patent Literature 1)
(2) WO 2018/199146 A (Patent Literature 2)
(3) WO 2018/199147 A (Patent Literature 3)
(4) WO 2019/208731 A (Patent Literature 4)
(5) WO 2021/085635 A (Patent Literature 5)
(6) WO 2021/085636 A (Patent Literature 6)
(7) WO 2021/149814 A (Patent Literature 7)
(8) WO 2022/190486 A (Patent Literature 8)
(9) Chem. Sci., (2022), Vol.13, pp.6309-6315 (Non-Patent Literature 6)

### EXAMPLES

The present invention will be described in more detail below with reference to examples. However, the present invention should in no way be bound by the following examples, and can be implemented in any form within the scope that does not depart from the purpose of the invention.

The reaction system shown in Figure 1 was constructed, and the synthesis of tripeptide H₂N-AA³-AA²-AA¹-OtBu (where AA¹, AA², and AA³ each represent an amino acid residue) was carried out according to the above synthesis procedure. As shown in Figure 1, in this example, a pentafluorophenyl (PFP) group was used as the T^{a} group in Formula (R1), an Fmoc group was used as the PG^{a} group, and a t-butyl (tBu) group was used as the T^{b} group in Formula (R2). However, the present invention is not limited to the example and the form shown in the figure.

The following parts and equipment were used to construct the reaction system.
*Path: PTFE tube, diameter 0.1 cm
*DBU polymer: 595128, manufactured by Sigma-Aldrich Co. LLC
*Cartridge: 278800, manufactured by Tokyo Rika Kikai Co., Ltd., diameter 1.02 cm, length 10 cm or 30 cm
*Pump: TR-278550, manufactured by Tokyo Rika Kikai Co., Ltd.
*Temperature control device: MCR-1000, manufactured by Tokyo Rika Kikai Co., Ltd., product code 267950

In the following description, "reaction time" refers to, in the case of the amide bond formation reaction in step (i), the time from when the reaction system flows into the flow machine until immediately before it enters the deprotection cartridge, and in the case of the deprotection reaction in step (ii), the time from when the reaction system flows into the deprotection cartridge until when it flows out.

A 1.25 mL solution (concentration 0.1 M) of pentafluorophenyl ester of an amino acid whose N-terminal was protected with Fmoc (Fmoc-AA²-OPfp) (0.125 mmol) in tetrahydrofuran (THF) and a 1.25 mL THF solution (concentration 0.1 M) of a tert-butyl ester of another amino acid (H-AA¹-OtBu) (concentration 0.1M) were pumped (flow rate 1.0mL/min) and merged inside the pathway to carry out the amide bond formation reaction in step (i) (reaction temperature: room temperature, reaction time: <5 minutes), whereby an N-terminal protected dipeptide ester (Fmoc-AA²-AA¹-OtBu) was obtained. The resulting N-terminal protected dipeptide ester (Fmoc-AA²-AA¹-OtBu) was then pumped (flow rate 2.0 mL/min) through a cartridge (length 1 0 cm) to carry out the deprotection reaction of step (ii) (reaction temperature: 50°C, reaction time: <5 minutes), whereby a dipeptide ester (H₂N-AA²-AA¹-OtBu) with the N-terminus deprotected was obtained.

A THF solution (concentration 0.05 M) of the resulting dipeptide ester (H₂N-AA²-AA¹-OtBu) and a THF solution (concentration 0.05 M) of pentafluorophenyl ester of another amino acid with the N-terminus protected by Fmoc (Fmoc-AA³-OPfp) (concentration 0.05 M) were pumped (flow rate 1.0 mL/min) and merged inside the channel to thereby carry out the amide bond formation reaction in step (i), whereby a N-terminal protected tripeptide ester (Fmoc-AA³-AA²-AA¹-OtBu) was produced.

The above synthesis procedure was carried out while the amino acids AA¹, AA² and AA³ were varied to synthesize various N-terminal protected tripeptides Fmoc-H₂N-AA³-AA²-AA¹-OtBu. The results are shown in the table below. The "quant." in the table refers to substantially equivalent (i.e., approximately 100%).

**[Table 3]**

| No. | AA¹ | AA² | AA³ | Fmoc-AA³-AA²-AA¹-OtBu | Yield |
|---|---|---|---|---|---|
| 1 | L-Ala | L-Ala | L-Ala | Fmoc-L-Ala-L-Ala-L-Ala-O-tBu | quant. |
| 2 | L-Leu | L-Ala | L-Ala | Fmoc-L-Ala-L-Ala-L-Leu-O-tBu | quant. |
| 3 | L-Ile | L-Ala | L-Ala | Fmoc-L-Ala-L-Ala-L-Ile-O-tBu | quant. |
| 4 | L-Met | L-Ala | L-Ala | Fmoc-L-Ala-L-Ala-L-Met-O-tBu | 87% |
| 5 | L-Glu(tBu) | L-Ala | L-Ala | Fmoc-L-Ala-L-Ala-L-Glu(tBu)-O-tBu | 94% |
| 6 | L-Lys(Cbz) | L-Ala | L-Ala | Fmoc-L-Ala-L-Ala-L-Lys(Cbz)-O-tBu | 89% |
| 7 | L-Phg | L-Ala | L-Ala | Fmoc-L-Ala-L-Ala-L-Phg-O-tBu | 91% |
| 8 | L-Ala | L-Phe | L-Ala | Fmoc-L-Ala-L-Phe-L-Ala-O-tBu | 92% |
| 9 | L-Ala | L-Ala | L-Phe | Fmoc-L-Phe-L-Ala-L-Ala-O-tBu | 94% |

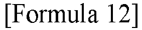

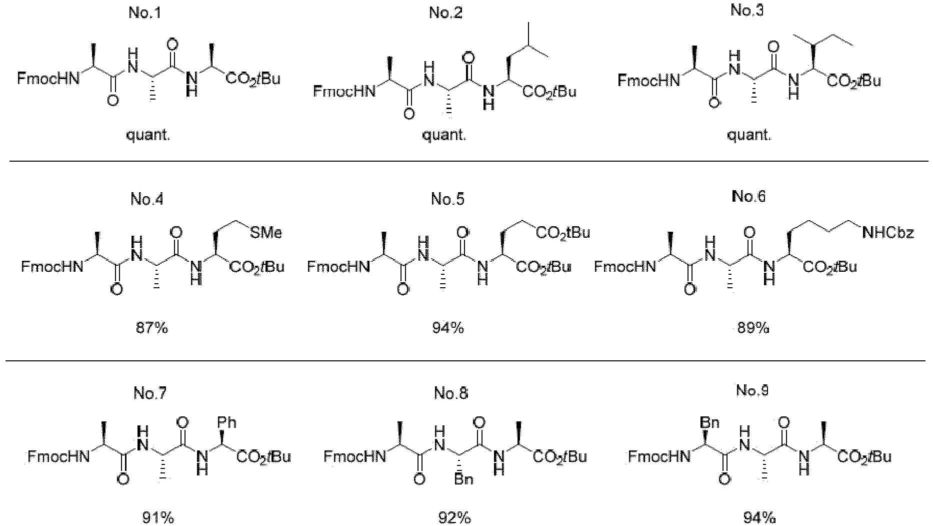

These N-terminal protected tripeptides, Fmoc-AA³-AA²-AA¹-OtBu, were circulated through a cartridge filled with DBU polymer using the same procedure as described above and pumped (flow rate 1.0 mL/min) through a cartridge filled with DBU polymer (length 30 cm) to carry out the deprotection reaction in step (ii). This allows the corresponding tripeptide ester (H₂N-AA³-AA²-AA¹-OtBu) to be obtained with the N-terminus deprotected.

In addition, the tripeptide ester H₂N-AA³-AA²-AA¹-OtBu synthesized using the above procedure was linked to another amino acid residue, alanine, as AA⁴ according to the following procedure to thereby produce a tetrapeptide. Specifically, a THF solution (concentration 0.05 M) of the tripeptide ester H₂N-AA³-AA²-AA¹-OtBu synthesized using the above procedure and a THF solution (concentration 0.05 M) of a pentafluorophenyl ester (Fmoc-AA⁴-OPfp) are each pumped (flow rate 1.0 mL/min) and merged inside the channel to carry out the amide bond formation reaction in step (i). Using the above procedure, the N-terminal protected tetrapeptide ester Fmoc-L-Ala-L-Ala-L-Ala-L-Ala-OtBu (i.e., the amino acid residues AA¹, AA², AA³ and AA⁴ are all L-alanine) was successfully synthesized (yield 92%).

## Claims

1. A method for producing a polypeptide compound, comprising:
(i) forming an amide bond between the substituted carboxyl group on the right side of an N-terminal-protected amino acid ester or peptide ester compound represented by formula (R1) and the amino group on the left side of an amino acid or peptide or amino acid ester or peptide ester compound represented by formula (R2) to thereby produce an N-terminal-protected peptide compound represented by formula (S1);
(ii) deprotecting the N-terminal of the compound of formula (S1) from step (i) to thereby produce a peptide compound represented by formula (P1); and
(iii) repeating steps (i) and (ii) with using the compound of formula (P1) from step (ii) as a compound of formula (R2) of step (i) to thereby extend the peptide chain via amidation. 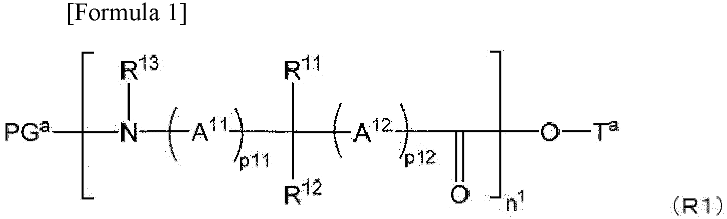 In formula (R1),
T^{a} represents a monovalent aromatic hydrocarbon group or heterocyclic group having one or more electron-withdrawing substituents,
PG^{a} represents a monovalent protecting group,
R¹¹and R¹² each represent, independently of each other, a hydrogen atom, halogen atom,
hydroxyl group, carboxyl group, nitro group, cyano group, or thiol group, or thiol group, or
an amino group, monovalent aliphatic hydrocarbon group, monovalent aromatic hydrocarbon group, or monovalent heterocyclic group that may have one or more substituents,
R¹³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
R¹¹ and R¹³ may be bound to each other to form, together with the carbon atom to which R¹¹ binds and the nitrogen atom to which R¹³ binds, a heterocyclic group that may have one or more substituents,
A¹¹ and A¹² each represent, independently of each other, a divalent aliphatic hydrocarbon group that may have one or more substituents having 1 to 3 carbon atoms,
p11 and p12 each represent, independently of each other, 0 or 1, and
n¹ represents an integer of equal to or greater than 1 corresponding to the number of the structure units parenthesized with [ ], provided that when n¹ is equal to or greater than 2, then the two or more structure units in [ ] may be either identical to each other or different from each other. 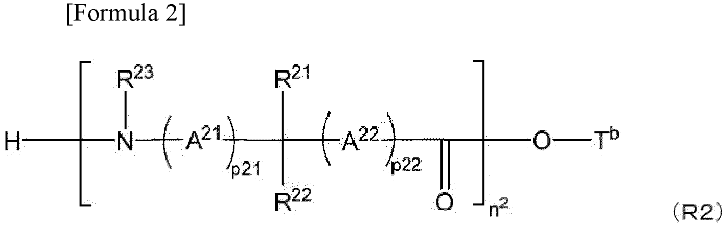
In formula (R2),
T^{b} represents a hydrogen atom or monovalent substituent,
R²¹and R²² each represent, independently of each other, a hydrogen atom, halogen atom,
hydroxyl group, carboxyl group, nitro group, cyano group, or thiol group, or thiol group, or an amino group, monovalent aliphatic hydrocarbon group, monovalent aromatic hydrocarbon group, or monovalent heterocyclic group that may have one or more substituents,
R²³ represents a hydrogen atom, carboxyl group, hydroxyl group, or a monovalent aliphatic hydrocarbon group, aromatic hydrocarbon group, or heterocyclic group that may have one or more substituents and may be bound to the nitrogen atom via a linking group, or
R²¹ and R²³ may be bound to each other to form, together with the carbon atom to which R²¹ binds and the nitrogen atom to which R²³ binds, a heterocyclic group that may have one or more substituents,
A²¹ and A²² each represent, independently of each other, a divalent aliphatic hydrocarbon group that may have one or more substituents having 1 to 3 carbon atoms,
p21 and p22 each represent, independently of each other, 0 or 1, and
n² represents an integer of equal to or greater than 1 corresponding to the number of the structure units parenthesized with [ ], provided that when n² is equal to or greater than 2,
then the two or more structure units in [ ] may be either identical to each other or different from each other. 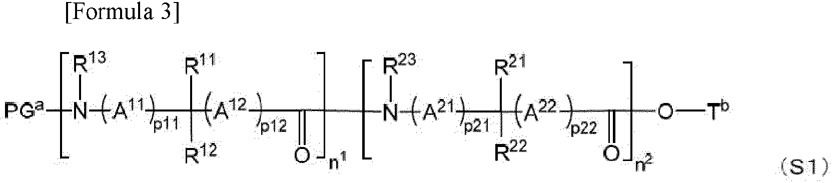
In formula (S1),
PG^{a}, R¹¹, R¹², R¹³, A¹¹, A¹², p11, p12, and n¹ each represent the same definitions as those of the same symbols in general formula (R1), and
R²¹, R²², R²³, A²¹, A²², p21, p22, n², and T^{b} each represent the same definitions as those of the same symbols in general formula (R2). 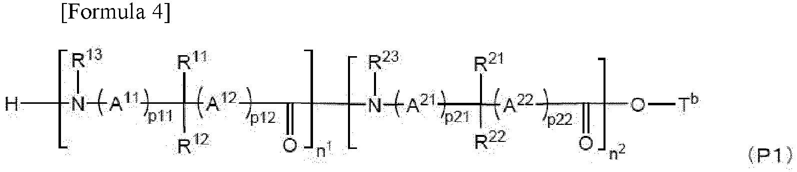
In formula (P1),
R¹¹, R¹², R¹³, A¹¹, A¹², p11, p12, and n¹ each represent the same definitions as those of the same symbols in general formula (R1), and
R²¹, R²², R²³, A²¹, A²², p21, p22, n², and T^{b} each represent the same definitions as those of the same symbols in general formula (R2).

2. The method according to claim 1, wherein steps (i) and (ii) are carried out continuously as a flow reaction.

3. The method according to claim 1 or 2, wherein in the reaction of step (i), the compound of formula (R1) and the compound of formula (R2) are used at approximately equal mole ratios.

4. The method according to any one of claims 1 to 3, wherein the substituent T^{a} of formula (R1) is an aromatic hydrocarbon group having one or more substituents selected from a halogen atom, halogen substitution alkyl group, halogen substitution alkoxy group, nitro group, acetyl group, ester group, sulfonic acid ester group, and amide group.

5. The method according to any one of claims 1 to 4, wherein the protecting group PG^{a} of formula (R1) is a monovalent hydrocarbon group, acyl group, hydrocarbon oxycarbonyl group, hydrocarbon sulfonyl group, and amide group that may have one or more substituents.

6. The method according to any one of claims 1 to 5, wherein deprotection of the N-terminal-protected group in formula (S1) at step (ii) is carried out by making the compound of formula (S1) pass through a column filled with basic ion exchange resin.

7. The method according to claim 6, wherein the basic ion exchange resin is selected from 1,8-diazabicyclo[5.4.0]undec-7-ene (DBU) resin, 1,5-diazabicyclo[4.3.0]non-5-ene (DBN) resin, 1,5,7-triazabicyclo[4.4.0]dec-5-ene (TBD) resin, piperazine resin, dimethylaminopyridine resin, and ammonium resin.
